# EUROPEAN PATENT APPLICATION

(11) **EP 4 156 205 A1**
(43) Date of publication of application: **29.03.2023**
(21) Application number: 22763227.0
(22) Date of filing: 01.03.2022
(51) Int. Cl.: G16H 80/00

(54) **INFORMATION PROVISION SYSTEM, MANAGEMENT SERVER, PROGRAM, AND STORAGE MEDIUM**

(30) Priority: 03.03.2021 JP 2021033692
(71) Applicant: Universal Training Center, Inc., Tokyo 150-0043 (JP)
(72) Inventor: SUGAWARA, Mizuki, Tokyo 150-0043 (JP)
(74) Representative: Marks & Clerk LLP
(86) International application number: PCT/JP2022/008487
(87) International publication number: WO 2022/186172

(57) **Abstract**

An object to be attained by the present invention is to convey information to a seat user in real time. An information providing system of the present invention is a system including a management server provided in a cloud, a sensing device, a seat user terminal, and an advice provider terminal, which are connected to one another via a communication network. The management server includes a communication part and an advice reception part. Advice is received and is then transmitted to the seat user terminal. The management server of the present invention includes a communication part and an advice reception part. Advice is received and is then transmitted to the seat user terminal. A program of the present invention is a program for causing a computer to function as the management server. A storage medium of the present invention is a computer-readable storage medium having the program recorded thereon.

## Description

### Technical Field

The present invention relates to an information providing system for providing information to a user of a wheelchair, a seat in a vehicle, or various other seats, and to a management server, a program, and a storage medium that are usable to build the information providing system.

### Background Art

To sit in a wheelchair, a seat in a vehicle, or various other seats for a long stretch of time is said to lead to various health risks. For example, many of wheelchair users who spend a long time on a wheelchair suffer from a pressure sore on buttocks caused by a gap in seated body pressure or concentration of a shearing force on buttocks. In addition, not a small number of people who sit on a chair or other seats for a long stretch of time, such as people who drive a vehicle for long hours and people who work long hours at a desk, suffer from low back pain, stiff shoulder, and other kinds of unwellness.

Hitherto, as a system for monitoring a seated posture on a seat, there has been proposed a monitoring system including a pressure distribution sensor, which detects distributions of pressure acting on a seat surface and a seat back of a chair, converts detected distributions into electric signals, and outputs the electric signals, and a posture estimation apparatus, which estimates a seated posture based on pressure information obtained from the pressure distribution sensor (Patent Literature 1).

This monitoring system is configured to issue, by utilizing a fact that a position or a tilt of a center-of gravity line changes in response to a change in seated posture of a wheelchair user, alert informing that assistance from a caretaker is required when a state in which the position or the tilt of the center-of-gravity line exceeds a threshold value set in advance lasts for a predetermined length of time.

### Citation List

### Patent Literature

[PTL 1] JP 2018-102861 A

### Summary of Invention

### Technical Problem

In Patent Literature 1, the wheelchair user is informed by the notification that assistance from a caretaker is required, but there is no instruction on what action is to be taken by the wheelchair user himself or herself. It is accordingly difficult for the wheelchair user to take an appropriate response himself or herself in real time, and the wheelchair user may have no choice but wait for a caretaker to arrive.

The present invention has been made in view of the circumstance as described above, and an object to be attained by the present invention is to provide an information providing system capable of conveying information to a seat user in real time, and a management server, a program, and a storage medium that are usable to build the information providing system.

The term "real time" in this application is not limited to simultaneity in a strict sense, and is a concept that includes a time spent for various types of processing, such as data communication, analysis, and recording, and that includes approximately several seconds to several ten seconds after acquisition of data.

### Solution to Problem

An information providing system according to one embodiment of the present invention is a system including a management server provided in a cloud, a sensing device configured to acquire data about a seating situation of a seat user and transmit the data to the management server, a seat user terminal to be used by the seat user, and an advice provider terminal to be used by an advice provider, the management server, the sensing device, the seat user terminal, and the advice provider terminal being connected to one another via a communication network. The management server includes a communication part configured to hold data communication via the communication network, and an advice reception part configured to receive advice transmitted from the advice provider terminal. The advice is configured to be received by the advice reception part and then transmitted to the seat user terminal via the communication part. A management server according to one embodiment of the present invention is a management server provided in a cloud to provide information to a seat user via a communication network, and the management server includes: a communication part configured to hold data communication via the communication network; and an advice reception part configured to receive advice transmitted from an advice provider terminal, which is connected via the communication network. The advice is configured to be received by the advice reception part and then transmitted, via the communication part, to a seat user terminal connected via the communication network. A program according to one embodiment of the present invention is a program for causing a computer to function as the management server. A storage medium according to one embodiment of the present invention is a computer-readable storage medium having the program recorded thereon.

### Advantageous Effects of Invention

The information providing system according to the present invention is configured to transmit advice provided with use of the advice provider terminal to the seat user terminal, and, accordingly, the seat user viewing the advice can immediately take a specific response. The same effect is obtained from the management server, the program, and the storage medium according to the present invention as well.

### Brief Description of Drawings

FIG. 1 is an overall configuration diagram of a pressure sore prevention system.
FIG. 2 is an explanatory diagram for illustrating an outline of an installation-type sensing device (seating device).
FIG. 3(a) is an explanatory diagram for illustrating arrangement of seat surface sensors, and FIG. 3(b) is an explanatory diagram for illustrating arrangement of seat back sensors.
FIG. 4 is an explanatory diagram for illustrating configurations of a control unit and a sensor unit.
FIG. 5 is a hardware configuration diagram of a management server.
FIG. 6 is a diagram for illustrating a configuration of the management server.
FIG. 7(a) is an explanatory diagram for illustrating an example of a table prepared in a user information storage part, FIG. 7(b) is an explanatory diagram for illustrating an example of a table prepared in a sensor information storage part, and FIG. 7(c) is an explanatory diagram for illustrating an example of a table prepared in an alert information storage part.
FIG. 8 is a hardware configuration diagram of a user terminal.
FIG. 9 is a sequence diagram of a case of logging in with use of the user terminal.
FIG. 10(a) is an explanatory diagram for illustrating an example of a login screen, and FIG. 10(b) is an explanatory diagram for illustrating an example of a main menu to be displayed on a main screen.
FIG. 11 is a sequence diagram of a case of user registration that uses the user terminal.
FIG. 12 is an explanatory diagram for illustrating an example of a user information registration screen.
FIG. 13 is a sequence diagram of a case of referring to data with the use of the user terminal.
FIG. 14(a) is an explanatory diagram for illustrating an example of a data reference screen, and FIG. 14(b) is an explanatory diagram of a seat surface heat map and a shearing force map.
FIG. 15 is a sequence diagram of a case of displaying, from the data reference screen displayed on the user terminal, a data reference screen in a "period specified" mode.
FIG. 16(a) is an explanatory diagram for illustrating an example of the data reference screen in the "period specified" mode, and FIG. 16(b) is an explanatory diagram for illustrating an example of a bodily movement item list displayed on the data reference screen of FIG. 16(a).
FIG. 17 is a sequence diagram of a case of executing action inquiry with the use of the user terminal.
FIG. 18 is an explanatory diagram for illustrating an example of an action inquiry screen.
FIG. 19 is a sequence diagram of a case of setting various settings with the use of the user terminal.
FIG. 20 is an explanatory diagram for illustrating an example of a setting screen.
FIG. 21 is a sequence diagram of a case of referring to an advice list with the use of the user terminal.
FIG. 22 is an explanatory diagram for illustrating an example of an advice list screen.
FIG. 23 is a sequence diagram of a case of issuing an inquiry about a specific piece of advice with the use of the user terminal.
FIG. 24 is an explanatory diagram for illustrating an example of an advice inquiry screen.
FIG. 25 is a sequence diagram of a case of issuing an inquiry about details of a specific piece of alert from the advice inquiry screen displayed on the user terminal.
FIG. 26 is a sequence diagram of a case of issuing an inquiry about details of a specific piece of alert from the advice list screen displayed on the user terminal.
FIG. 27 is a sequence diagram of a case of deleting a specific piece of advice from the advice list screen displayed on the user terminal.
FIG. 28 is a sequence diagram of a case of newly registering advice through the user terminal.
FIG. 29(a) is an explanatory diagram for illustrating an example of a new advice input screen, and FIG. 29(b) is an explanatory diagram for illustrating the example of the new advice input screen.
FIG. 30 is a sequence diagram of a case of referring to an alert list from the main screen displayed on the user terminal.
FIG. 31(a) is an explanatory diagram for illustrating an example of an alert list screen, and FIG. 31(b) is an explanatory diagram for illustrating an example of an alert inquiry screen.
FIG. 32 is a sequence diagram of a case of issuing an inquiry about details of a specific piece of alert from the alert list screen displayed on the user terminal.
FIG. 33 is a sequence diagram of a case of referring to, from the alert inquiry screen displayed on the user terminal, advice related to a piece of alert of the alert inquiry screen.
FIG. 34 is an explanatory diagram for illustrating details of a pressure sensor that uses conductive threads.
FIG. 35(a) is an explanatory diagram for illustrating an example of a bottom wear-side wearable piece, and FIG. 35(b) is an explanatory diagram for illustrating an example of a top wear-side wearable piece.

### Description of Embodiments

### (Embodiment)

An example of an embodiment of the present invention is described with reference to the drawings. The present invention is applicable to various uses. Here, a case of applying the present invention to a system for preventing a pressure sore of a person who uses a wheelchair (seat) X (hereinafter referred to as "wheelchair user") (the system is hereinafter referred to as "pressure sore prevention system") is taken as an example.

### [Outline of Pressure Sore Prevention System]

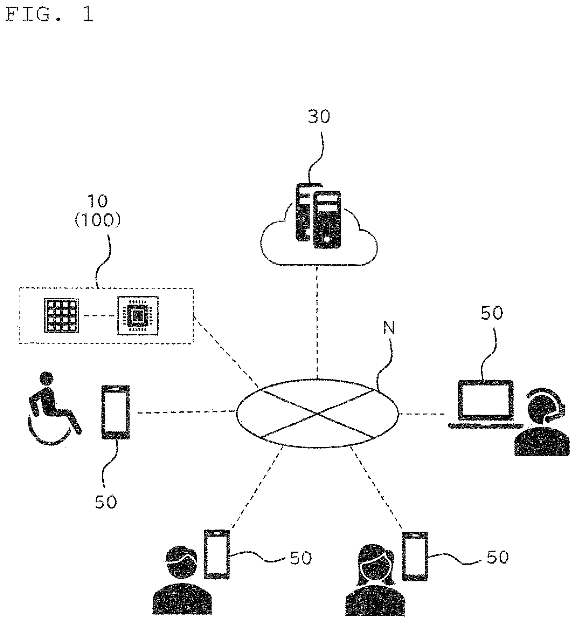

An overall configuration of the pressure sore prevention system of this embodiment is illustrated in FIG. 1. As illustrated in FIG. 1, the pressure sore prevention system of this embodiment includes a seating device 10, a management server 30, and a user terminal 50 used by each user. The seating device 10 and each user terminal 50 are connected so that communication to and from the management server 30 can be held via a communication network N.

In this embodiment, wheelchair users, physical therapists, care workers (helpers), family members, medical service workers (medical doctors and nurses), insurance service personnel, care managers, welfare equipment advisors, local governments, and the like are assumed to be users. For the sake of convenience of description, the user terminal 50 used by a wheelchair user and the user terminal 50 used by a person who provides advice may be referred to as "sheet user terminal" or "wheelchair user terminal" and "advice provider terminal," respectively, in the following description.

### [Seating Device]

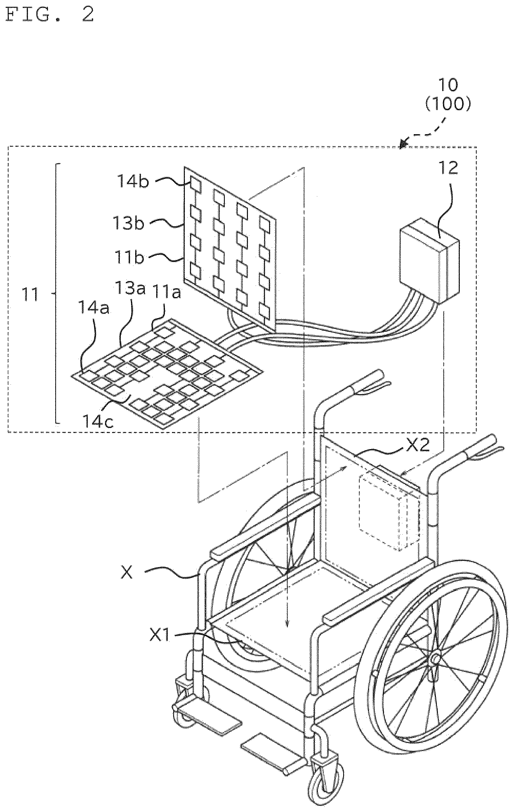

The seating device 10 in the pressure sore prevention system of this embodiment is described first with reference to the drawings. The seating device 10 in this embodiment is an installation-type sensing device 100 (hereinafter referred to as "installation-type device") to be used installed in a wheelchair X. The seating device 10 illustrated in FIG. 2 as an example includes a sensor unit 11 and a control unit 12. The sensor unit 11 is placed on a seat surface X1 of the wheel chair X, and a front side of a back support X2 of the wheel chair X. The control unit 12 is attached to any place on the wheelchair X, for example, a back side of the back support X2, a side surface, a seat underside, or a bottom net. The control unit 12 is adjustable to a size suitable for the place to which the control unit 12 is attached.

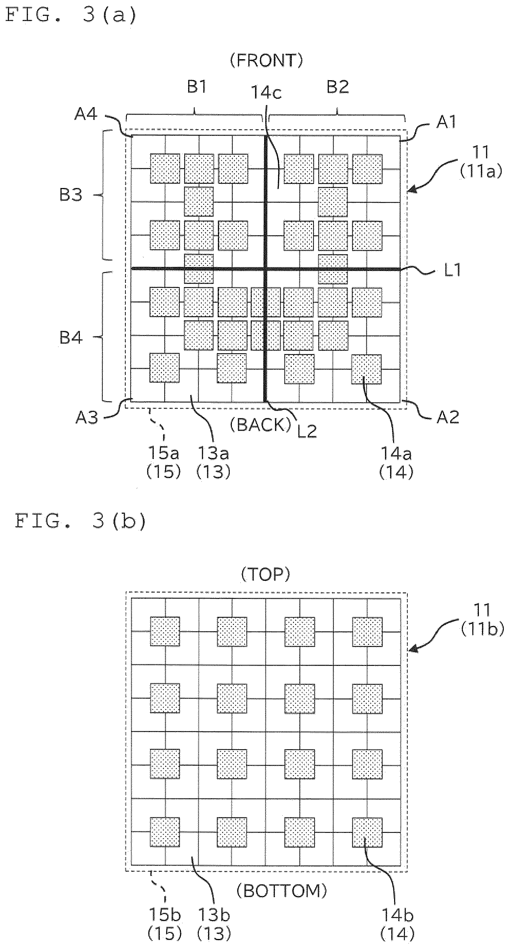

The sensor unit 11 illustrated in FIG. 2, FIG. 3(a), and FIG. 3(b) as an example includes sensor sheets 13, sensors 14, and seat covers 15. The sensor unit 11 in this embodiment includes a seat surface part 11a placed on the seat surface X1 of the wheelchair X to detect pressure applied to the seat surface X1, and a seat back part 11b placed on the front side of the back support X2 of the wheelchair X to detect pressure applied to the back support X2.

The sensor sheets 13 are sheets for affixing the sensors 14 thereto. The sensor sheets 13 in this embodiment include a seat surface sheet 13a and a seat back sheet 13b. Various materials are usable for the sensor sheets 13. In this embodiment, sheets made of a material having flexibility and a waterproof property are used. Although the seat surface sheet 13a and the seat back sheet 13b are configured as separate components in this embodiment, the two may be configured as an integrated component.

The sensors 14 detect pressure applied to the buttocks and the back of a seated wheelchair user and convert the pressure into electric signals. Pressure sensors, load vector sensors, or the like are usable for the sensors 14. In this embodiment, a pressure sensor having an effective sensor area of 39.6 mm × 39.6 mm, a thickness of from 0.20 mm to 1.25 mm, a pressure sensing range of from 0.2 N to 20 N, and a minimum sensitivity of from 20 g to 100 g is used as each of the sensors 14. This pressure sensor is a type of polymer thick film, and decreases in resistance value when pressure (a load) is applied to a sensor part. Resistance characteristics of this pressure sensor expressed in the form of a graph are a curve indicating a gradual decrease of the resistance value that accompanies an increase in load.

The sensors 14 are placed at individual measurement points as independent sensors. The control unit 12 calculates a load (pressure) applied to each one of the sensors 14 by inverse operation from an electric resistance measured by the one of the sensors 14. The use of this type of pressure sensor as each one of the sensors 14 enables specification of pressure applied to the one of the sensors 14 as an absolute value, irrespective of a measurement value of another of the sensors 14.

As illustrated in FIG. 2 to FIG. 4, in this embodiment, the seat surface sheet 13a is provided with thirty-two sensors (hereinafter referred to as "seat surface sensors") 14a, and the seat back sheet 13b is provided with sixteen sensors (hereinafter referred to as "seat back sensors") 14b.

The thirty-two seat surface sensors 14a provided on the seat surface sheet 13a are placed in a substantially U-shaped pattern in plan view in portions in which the left and right thighs of the wheelchair user are located (femoral areas) and portions in which the buttocks of the wheelchair user are located (a gluteal area). Each of portions corresponding to two arm portions of the U shape forms one femoral area, and a portion corresponding to a joint portion (a portion linking the two arm portions) of the U shape forms the gluteal area. The seat surface sheet 13a is provided with a no-sensor area 14c in which no seat surface sensors 14a are installed.

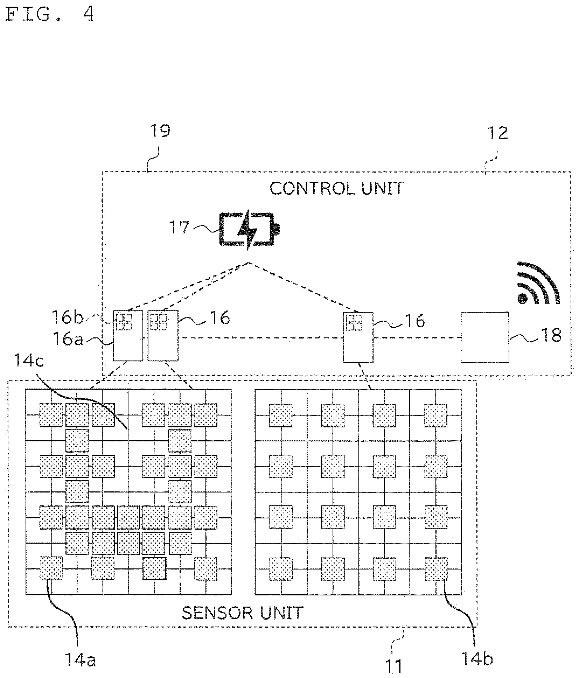

The no-sensor area 14c here is an area that is a target of interpolation processing in display of a seat surface heat map 81 described later. If sensors that are the same as the seat surface sensors 14a are to be placed in the no-sensor area 14c, the no-sensor area 14c includes an area surrounded by actually installed seat surface sensors 14a in, out of eight directions (upper, lower, left, right, upper right, lower right, upper left, and lower left directions in FIG. 3(a) and FIG. 4) of the no-sensor area 14c, (A) two or more directions out of four directions that are the upper, lower, left, and right directions, (B) one direction out of four directions that are the upper, lower, left, and right directions, and two or more directions out of the upper right, lower right, upper left, and lower left directions, or (C) three or more directions out of the upper right, lower right, upper left, and lower left directions.

The provision of the no-sensor area 14c in which no seat surface sensors 14a are placed on the seat surface sheet 13a (seat surface part 11a) minimizes the number of seat surface sensors 14a. As a result, a time required to transmit data to the management server 30 (external equipment) can be shortened, and a system capable of conveying information to the wheelchair user in real time can be built.

Specifically, sensors in each femoral area are arranged so that three sensors, one sensor, three sensors, and one sensor are aligned from the front side to the back side, and sensors in the gluteal area are arranged so that seven sensors, five sensors, and four sensors are aligned from the front side to the back side.

Each femoral area has a portion in which the seat surface sensors 14a are arranged in a fishbone pattern by staggering, in a front-back direction, a portion in a larger number of (three in the example of the drawings) seat surface sensors 14a are placed in a left-right direction and a portion in which a smaller number of (one in the example of the drawings) seat surface sensors 14a are placed in the left-right direction, such as placing three sensors in one portion, one sensor in the next portion, three through one sensor in a portion behind the next portion, then three sensors, and lastly one sensor.

The gluteal area does not have any no-sensor area 14c in a front half and, in a back half, has a portion including at least one no-sensor area 14c by placing, for example, seven seat surface sensors 14a in the front half and four seat surface sensors 14a in the back half.

The arrangement of the seat surface sensors 14a described here is an example, and the seat surface sensors 14a may be arranged in other patterns.

As illustrated in FIG. 2 to FIG. 4, the sixteen seat back sensors 14b provided on the seat back sheet 13b are arranged so that rows each including four sensors are placed at equal intervals from a top downward. The arrangement of the seat back sensors 14b described here is an example, and the seat back sensors 14b may be arranged in other patterns.

In the following description, the seat surface sheet 13a is divided into four areas compartmentalized by a front-back division line L1 dividing the seat surface sheet 13a into equal areas in the front-back direction, and a left-right division line L2 dividing the seat surface sheet 13a into equal areas in the left-right direction, as illustrated in FIG. 3(a), and an upper right area, a lower right area, a lower left area, and an upper left area are referred to as "first quadrant A1," "second quadrant A2," "third quadrant A3," and "fourth quadrant A4," respectively.

An area to the left of the left-right division line L2 of FIG. 3(a) (the third quadrant A3 and the fourth quadrant A4), an area to the right of the left-right division line L2 (the first quadrant A1 and the second quadrant A2), an area above the front-back division line L1 (the first quadrant A1 and the fourth quadrant A4), and an area below the front-back division line L1 (the second quadrant A2 and the third quadrant A3) are referred to as "left-side area B1," "right-side area B2," "front-side area B3," and "back-side area B4," respectively.

The seat covers 15 cover the sensor sheets 13 and the sensors 14 affixed to the sensor sheets 13. The seat covers 15 in this embodiment include a seat surface cover 15a for covering the seat surface sheet 13a and the seat surface sensors 14a, and a seat back cover 15b for covering the seat back sheet 13b and the seat back sensors 14b. Various materials are usable for the seat covers 15. In this embodiment, sheets made of a material having flexibility and a waterproof property are used.

The control unit 12 is a unit for controlling the sensor unit 11. As illustrated in FIG. 4, the control unit 12 in this embodiment includes microcomputers 16, a battery 17, and a communication module 18. The microcomputers 16, the battery 17, and the communication module 18 are housed in a storage box 19.

The microcomputers 16 process signals acquired from the seat surface sensors 14a and the seat back sensors 14b, and transmit the processed signals to the management server 30 via the communication module 18. The microcomputers 16 each include a microcomputer board 16a and various electronic parts 16b mounted on the microcomputer board 16a. For example, a product of Arduino is usable for the microcomputer board 16a.

The communication module 18 for transmitting data acquired by the sensors 14 to the management server 30 is connected to the microcomputers 16. For example, a 3G loT module (3GIM) is usable for the communication module 18.

A module having a function of the Global Positioning System (GPS) may also be used for the communication module 18. When the communication module 18 having a GPS function is used, data that can be acquired in addition to position information data of the wheelchair user, includes data about a travel speed, which is acquired from the position information and from a travel time. The GPS module may be provided separately from the communication module 18.

As illustrated in FIG. 4, in this embodiment, one microcomputer board 16a is connected per sixteen sensors 14. The sensors 14 are connected by wired connection to each microcomputer board 16a, and each microcomputer board 16a acquires data from sixteen sensors 14 allocated thereto. Serial communication is held between one microcomputer board 16a and another microcomputer board 16a to transmit data acquired by each microcomputer board 16a to the management server 30 via the shared communication module 18. Although a case of using a plurality of microcomputer boards 16a is discussed here as an example, a configuration in which all sensors 14 are connected to one microcomputer board 16a is also employable.

The shared battery 17 is connected to each microcomputer board 16a so that power is supplied to each microcomputer board 16a from the battery 17. An existing lithium ion secondary battery or the like is usable for the battery 17. The battery 17 and each microcomputer board 16a are connected to each other inside the storage box 19.

The pressure sore prevention system of this embodiment is designed so that acquired data is transmitted to the management server 30 for every few seconds, from the viewpoint of conveying information in real time to the wheelchair user. The system may be modified so that the data is transmitted at intervals shorter than or longer than every few seconds. The transmitted data is processed on the management server 30. Processing on the management server 30 is described later.

The configuration of the seating device 10 described above is an example, and the seating device 10 may have other configurations as long as the desired object is attained.

### [Management Server]

Next, the management server 30 in the pressure sore prevention system of this embodiment is described with reference to the drawings. The management server 30 is a so-called cloud server (cloud system) which executes, among others, acquisition, processing, aggregation, recording, and update of data transmitted from the seating device 10, and response to a request from each user terminal 50.

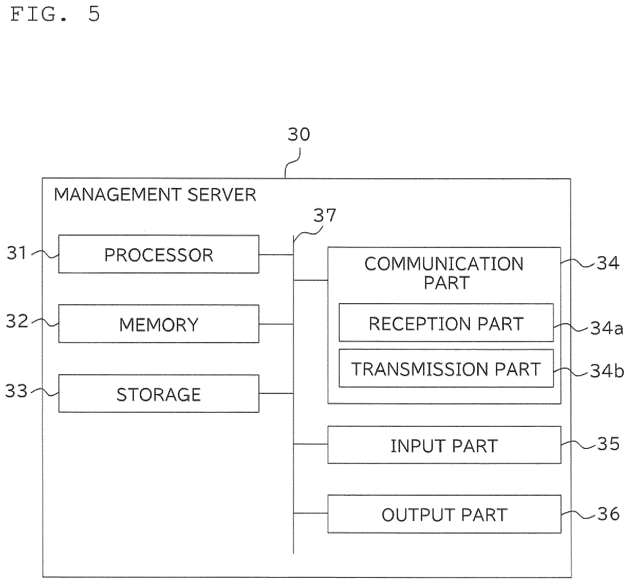

As illustrated in FIG. 5, the management server 30 of this embodiment includes, as main components, a processor 31, a memory 32, a storage 33, a communication part 34, an input part 35, and an output part 36. Those components are electrically connected to one another through a bus 37. Although omitted from the drawing, it is preferred for the management server 30 to have a configuration including a power source, a storage device, an uninterruptible power source, a backup device, and the like so as to stably provide a service to the plurality of user terminals 50.

The processor 31 executes overall control of the components connected by the bus 37, and includes, for example, a central processing unit (CPU), a micro-processing unit (MPU), or the like. The processor 31 executes each processing procedure by loading an application program required to execute the processing procedure from the storage 33 onto the memory 32.

The memory 32 is a main storage device for storing data and commands, and includes, for example, a read-only memory (ROM), a random access memory (RAM), or the like.

The storage 33 is an auxiliary storage device for storing programs and data, and includes, for example, a hard disk drive (HDD), a solid state drive (SSD), a flash memory, or the like. The storage 33 stores an application program of the pressure sore prevention system of this embodiment.

The communication part 34 is a communication interface for holding wireless communication (data communication) to and from the seating device 10 and each user terminal 50 via the communication network N. Communication through the communication part 34 is in conformity to TCP/IP and various other communication standards, such as Bluetooth Low Energy (trademark), Bluetooth (trademark), 3rd generation (3G), 4th generation (4G), and Long Term Evolution (LTE).

The communication part 34 includes a reception part 34a and a transmission part 34b. The reception part 34a receives a signal from each user terminal 50, decodes the received signal, and conveys the decoded signal to the processor 31. The transmission part 34b transmits information conveyed from the processor 31 to each user terminal 50.

The input part 35 is a device for inputting information, and includes, for example, one or more out of a keyboard, a touch screen, a mouse, and an audio input device. The output part 36 is a device for outputting information, and includes a display, a printer, and the like.

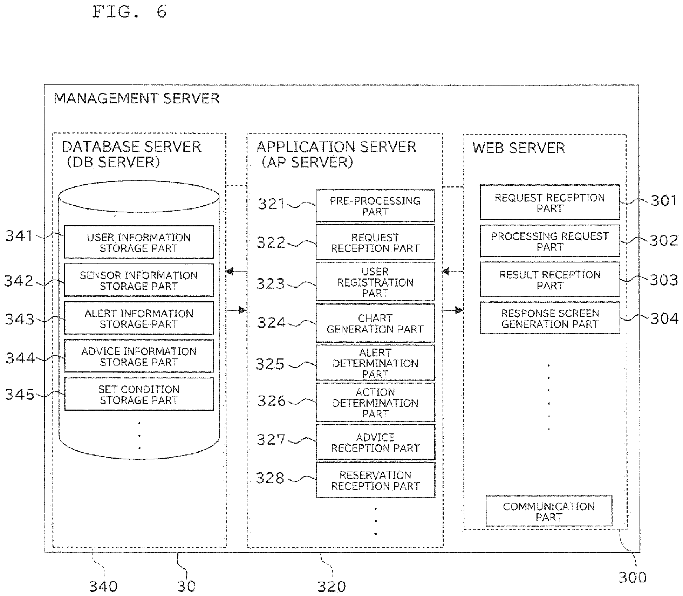

The management server 30 can be configured from a group of two or more servers. As illustrated in FIG. 6, the management server 30 of this embodiment includes a Web server 300, and application server (hereinafter referred to as "AP server") 320, and a database server (hereinafter referred to as "DB server") 340, which are connected to one another in a manner that enables communication to and from one another via a LAN.

The Web server 300 is a server for providing a Web service in response to a request from the user terminal 50. The Web server 300 in this embodiment includes, as main components, a request reception part 301, a processing request part 302, a result reception part 303, and a response screen generation part 304.

The request reception part 301 is a function part for receiving a request from the user terminal 50. The processing request part 302 is a function part for requesting the AP server 320 to execute processing. The result reception part 303 is a function part for receiving a processing result transmitted from the AP server 320. The response screen generation part 304 is a function part for generating a screen to be transmitted to the user terminal 50.

The AP server 320 is a server for executing an application program based on a request from the Web server 300. The AP server 320 in this embodiment includes, as main components, a pre-processing part 321, a request reception part 322, a user registration part 323, a chart generation part 324, an alert determination part 325, an action determination part 326, an advice reception part 327, and a reservation reception part 328.

The pre-processing part 321 is a function part for formatting data transmitted from the seating device 10. The request reception part 322 is a function part for receiving a request from the Web server 300. The user registration part 323 is a function part for executing processing of user registration. The chart generation part 324 is a function part for generating, in response to a request from the Web server 300, heat maps 81 and 82, a shearing force map 83, various lists, a graph, and the like. The alert determination part 325 is a function part for determining, based on data, whether an alert condition is satisfied. The advice reception part 327 is a function part for receiving new advice registration. The reservation reception part 328 is a function part for receiving a reservation for remote rehabilitation.

The pre-processing part 321 formats raw data (enumeration of numbers in which 4-second-worth of measurement data is compiled) acquired by the seating device 10 into second-by-second data, and, based on the data obtained by the formatting (hereinafter referred to as "formatted data"), aggregates the number of times of a bodily movement at 1-minute intervals. The formatted data and data obtained by the aggregation (hereinafter referred to as "aggregated data") are stored in a sensor information storage part 342 of the DB server 340.

The chart generation unit 324 generates the heat maps 81 and 82, the shearing force map 83, various lists, a graph, and the like. In this embodiment, the seat surface heat map 81 and a seat back heat map 82 are generated as heat maps.

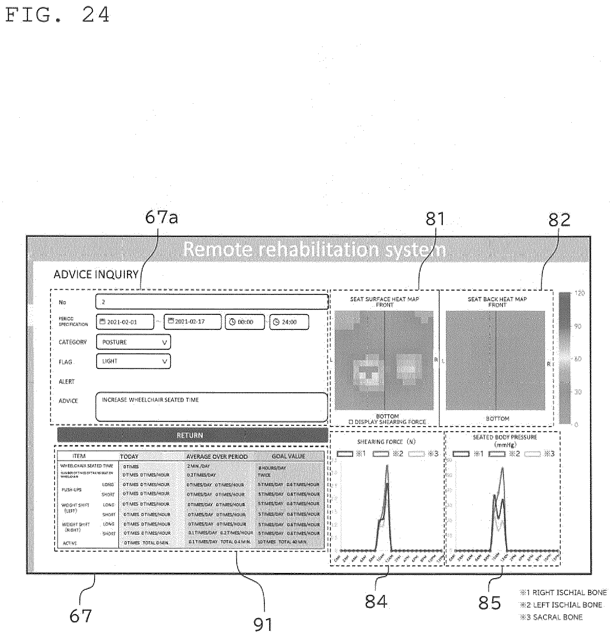

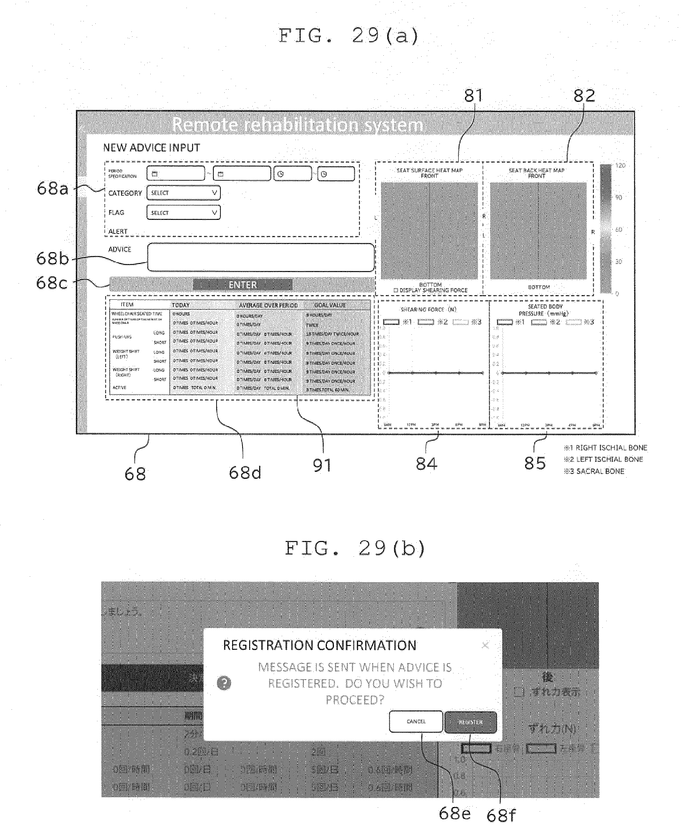

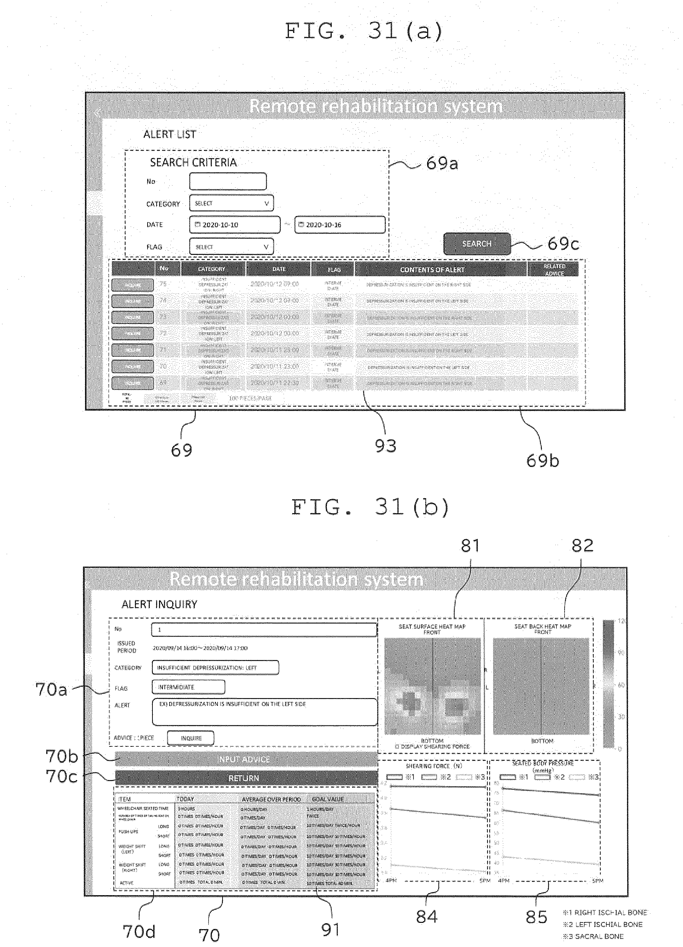

The seat surface heat map 81 displays, as pressure (seated body pressure) applied to the seat surface part 11a (seat surface X1), pressure values of the seat surface part 11a detected by the seat surface sensors 14a of the seating device 10 and interpolation values of the no-sensor area 14c calculated from the pressure values in colors that vary depending on magnitudes of those values. The seat surface heat map 81 is displayed on a data reference screen 63, an advice inquiry screen 67 (see FIG. 24), a new advice input screen 68 (see FIG. 29(a)), and an alert inquiry screen 70 (see FIG. 31(b)), which are described later. In this embodiment, seated body pressure in any area on the seat surface heat map 81 is displayed when the area is pointed by a cursor.

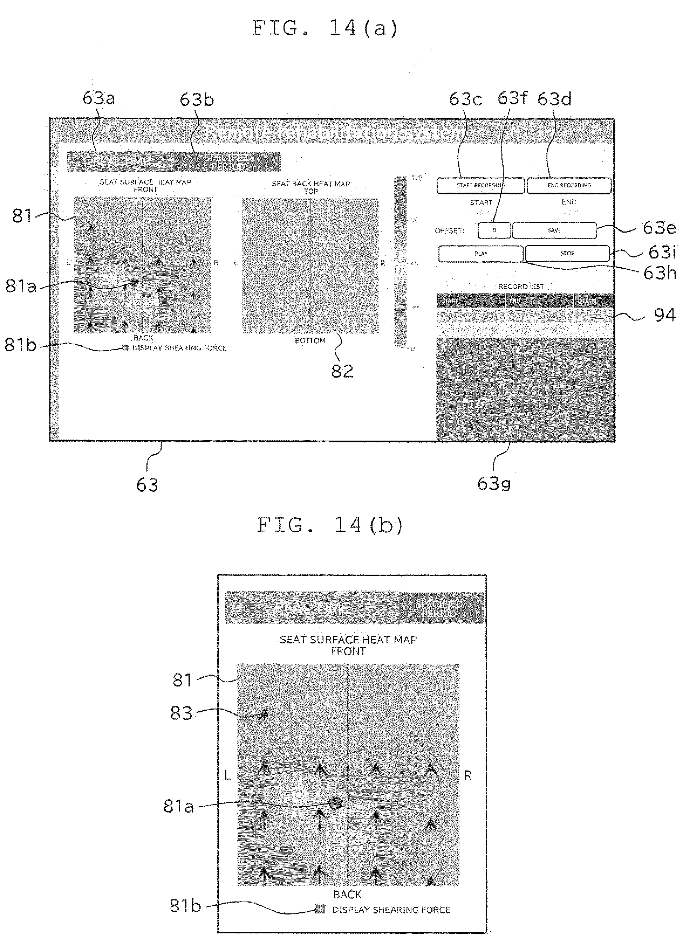

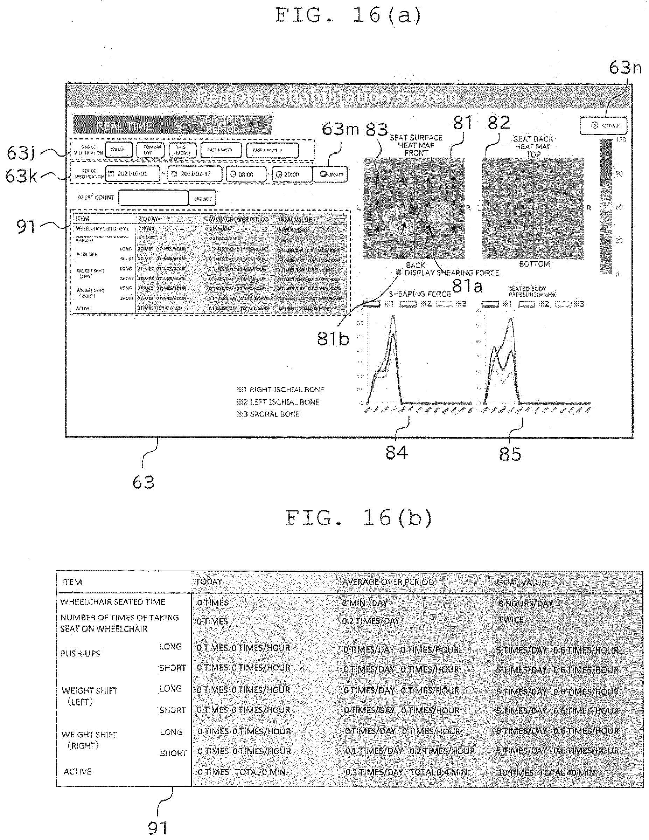

The seat back heat map 82 displays, as pressure applied to the seat back part 11b (back support X2), pressure values of the seat back part 11b detected by the seat back sensors 14b of the seating device 10 in colors that vary depending on the magnitudes of those values. The seat back heat map 82 is displayed on the data reference screen 63 (see FIG. 14(a) and FIG. 14(b), and FIG. 16(a) and FIG. 16(b)), the advice inquiry screen 67 (see FIG. 24), the new advice input screen 68 (see FIG. 29(a)), and the alert inquiry screen 70 (see FIG. 31(b)), which are described later. In this embodiment, seated body pressure in any area on the seat back heat map 82 is displayed when the area is pointed by the cursor.

The shearing force map 83 is a chart indicating a shearing force applied to the buttocks of the wheelchair user. In this embodiment, the shearing force is calculated from the pressure values of the seat surface part 11a acquired by the seat surface sensors 14a and the pressure values of the seat back part 11b acquired by the seat back sensors 14b. The shearing force is calculable by other methods as well.

A pressure sore on the back can be prevented by providing the seat back sensors 14b. When the wheelchair user repeats a bodily movement of reclining and depressurizing, friction against the back may lead to a laceration or a pressure sore. In this case, a pressure sore on the back is preventable by designing so that alert is issued when it is determined, from the pressure values of the seat back part 11b acquired by the seat back sensors 14b, that excessive pressure is being applied to the back.

The shearing force can be displayed by various methods. In this embodiment, as illustrated in FIG. 14(b), an arrow (vector) having a magnitude and a direction is displayed in a portion on which the shearing force is acting, and no vector is displayed in a portion on which the shearing force is not acting. In this embodiment, in addition to the display of the shearing force in the form of vectors, a numerical value of the shearing force in a portion in which an arrow is displayed is displayed, by pointing the cursor to the displayed arrow.

In this embodiment, the shearing force map 83 is displayed overlaid on the seat surface heat map 81 by checking a checkbox 81b of "display shearing force" displayed below the seat surface heat map 81, and stops being displayed by unchecking the checkbox 81b (see FIG. 14(b)). The shearing force map 83 is displayed on the data reference screen 63 (see FIG. 14(a) and FIG. 14(b), and FIG. 16(a) and FIG. 16(b)), the advice inquiry screen 67 (see FIG. 24), the new advice input screen 68 (see FIG. 29(a)), and the alert inquiry screen 70 (see FIG. 31(b)), which are described later.

The shearing force map 83 is also displayable on its own, without being overlaid on the seat surface heat map 81. Specifically, the shearing force map 83 may be displayed alone in an area in which the seat surface heat map 81 is displayed, without displaying the seat surface heat map 81.

In this embodiment, the chart generation part 324 generates, in addition to the heat maps 81 and 82 and the shearing force map 83, an advice list 92 in which pieces of advice are displayed in a list format, an alert list 93 in which pieces of alert are displayed in a list format, a record list 94 in which pieces of data recorded in the past are displayed in a list format, a bodily movement item list 91 in which pieces of information about bodily movement items are displayed in a list format, and others.

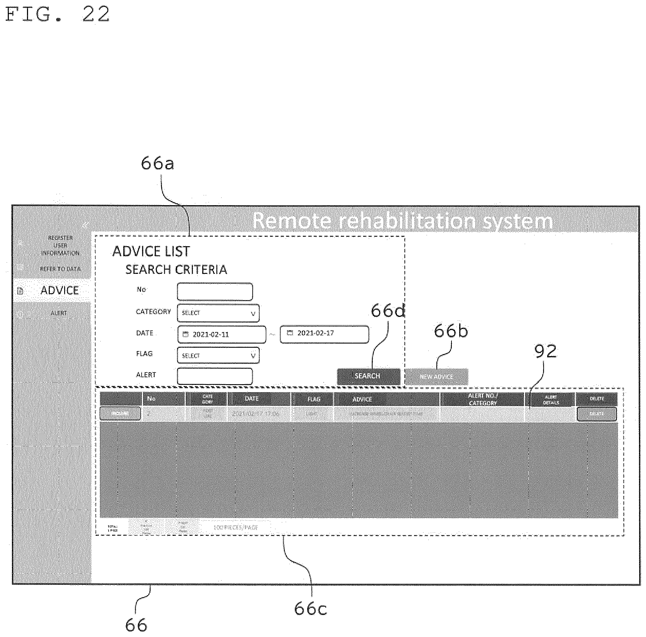

The advice list 92 includes, for example, an "inquiry field," a "No. field," a "category field," a "date field," a "flag field," an "advice field," an "alert No./category field," an "alert details field," and a "delete field." The advice list 92 is displayed on an advice list screen 66 (see FIG. 22) described later.

The alert list 93 includes, for example, an "inquiry field," a "No. field," a "category field," a "date field," a "flag field," an "alert contents field," and a "related advice field." The alert list 93 is displayed on an alert list screen 69 (see FIG. 31(a)) described later.

The bodily movement item list 91 displays a list of pieces of information that are the number of times of a bodily movement today, an average number of times of the bodily movement over a period, and a goal value of the number of times of the bodily movement. Bodily movement items include, for example, a wheelchair seated time, the number of times of taking a seat on the wheelchair, push-ups (long, short), weight shift left (long, short), weight shift right (long, short), and active. The bodily movement item list 91 is displayed on the data reference screen 63 in a "period specified" mode (see FIG. 16(a) and FIG. 16(b)), the advice inquiry screen 67 (see FIG. 24), the new advice input screen 68 (see FIG. 29(a)), and the alert inquiry screen 70 (see FIG. 31(b)), which are described later.

Other than those, the chart generation part 324 generates a shearing force graph 84, a seated body pressure graph 85, and an action graph 86. The shearing force graph 84 displays changes in shearing force at three specified points (a right ischial bone, a left ischial bone, and a sacral bone). The seated body pressure graph 85 displays changes in seated body pressure at the three specified points (the right ischial bone, the left ischial bone, and the sacral bone). The action graph 86 indicates the number of times and a duration of an action in a specified period.

The charts described here are an example, and the chart generation part 324 may generate charts other than those.

The alert determination part 325 determines whether data in a predetermined time satisfies an alert condition set in advance, generates alert when the data satisfies the alert condition, and transmits the alert to the Web server 300. In this embodiment, alert is transmitted to the wheelchair user terminal 50 via the communication part 34 when one of the following conditions is satisfied.

A first type of alert is high pressure alert. The high pressure alert is issued when a pressure average over a period of 30 minutes in one of quadrants from the first quadrant A1 to the fourth quadrant A4 exceeds a reference value set in advance.

For example, when it is determined in the alert determination part 325 that a pressure average over a period of 30 minutes in the first quadrant A1 exceeds a reference value set in advance, the alert determination part 325 generates high pressure alert to the effect that "a high pressure state of front right seated body pressure is detected," and transmits the high pressure alert to the Web server 300. The Web server 300 receives the high pressure alert, and transmits the high pressure alert to the user terminal 50 via the communication part 34.

A second type of alert is insufficient depressurization alert. The insufficient depressurization alert is issued when it is found out that depressurization falls short of a goal value set in advance as a result of searching an event log (push-ups and weight shifts) of depressurization of past 1 hour for every 30 minutes.

For example, when determining that the number of push-ups (long) on the right side in past 1 hour falls short of a goal value set in advance, the alert determination part 325 generates insufficient depressurization alert to the effect that "right depressurization is insufficient," and transmits the insufficient depressurization alert to the Web server 300. The Web server 300 receives the insufficient depressurization alert, and transmits the insufficient depressurization alert to the user terminal 50 via the communication part 34.

A third type of alert is high shearing force alert. The high shearing force alert is issued when a shearing force applied to the seat surface sensors 14a remains higher than a reference value set in advance for 30 minutes. In this embodiment, high shearing force alert is issued when a state in which the shearing force is higher than the reference value is observed even at one point.

For example, when it is determined in the alert determination part 325 that 30 minutes have elapsed with a pressure average in the first quadrant A1 staying higher than a reference value set in advance, the alert determination part 325 generates the high shearing force alert and transmits the high shearing force alert to the Web server 300. The Web server 300 receives the high shearing force alert, and transmits the high shearing force alert to the user terminal 50 via the communication part 34.

In this embodiment, alert is transmitted to an SNS installed in the user terminal 50 (viewable on the user terminal 50). With the proliferation of smartphones today, many people use SNSs as means of communication, and a configuration in which alert is transmitted to an SNS has accordingly a merit of increasing a chance of the alert being noticed by the wheelchair user and thus enabling the wheelchair user to take a quick response (action). However, alert may be transmitted by various other methods easy for the wheelchair user to check immediately (in a timely manner), such as an electronic mail and a short messaging service (SMS) viewable on the user terminal 50.

The action determination part 326 determines an action of the user based on a signal transmitted from the seating device 10. "Actions" here include, among others, a "wheelchair seated time," the "number of times of taking a seat on the wheelchair," "push-ups (long, short)," "weight shift left (long, short)," "weight shift right (long, short), and "active." The actions given here are an example, and actions other than those may be included.

Of the bodily movement items given above, the "wheelchair seated time" indicates a length of time for which the user is seated on the wheelchair X, and the "number of times of taking a seat on the wheelchair" indicates the total number of times the user has taken a seat on the wheelchair X. The action determination part 326 determines that the user has gotten on the wheelchair when a state in which an average value of the seated body pressure is equal to or higher than 10 mmHg lasts for 1 minute or longer, and starts counting. When a state in which an average value of the seated body pressure is less than 10 mmHg lasts for 1 minute or longer, the action determination part 326 determines that the user has gotten out of the wheelchair, and stops counting. In this case, a length of time from the point at which the user gets on the wheelchair to the point at which the user gets out of the wheelchair is obtained as the wheelchair seated time, and the number of times of getting on the wheel chair and getting out of the wheelchair is counted as the number of times of taking a seat on the wheelchair.

The term "push-ups" refers to a bodily movement of lifting buttocks with the body supported on hands for depressurization. The action determination part 326 counts a case in which the value in every one of the quadrants A1 to A4 remains 10 mmHg or less for 3 seconds or longer and shorter than 15 seconds as "short push-ups," and counts a case in which the value in every one of the quadrants A1 to A4 remains 10 mmHg or less for 15 seconds or longer and shorter than 60 seconds as "long push-ups."

The term "weight shift" refers to a bodily movement of tilting the body to the left or the right for depressurization of the buttocks one at a time. The action determination part 326 counts a case in which pressure applied to one of a left half and a right half of the seat surface X1 remains lower than a fixed value for 3 seconds to 15 seconds as a "short weight shift," and counts a case in which the pressure applied to one of the left half and the right half of the seat surface X1 remains lower than the fixed value for 15 seconds to 60 seconds as a "long weight shift."

The term "active" refers to a bodily movement of driving the wheelchair X or a state in which the user is exercising on the wheelchair X. The action determination part 326 determines that the user is "active" when the center of gravity (the center of pressure: COP) moves 10 cm or a longer distance in 5 seconds. Traveling on the wheelchair includes a time for waiting for a traffic light to change, and a time in which few bodily movements are performed in a course of the bodily movement of driving the wheelchair. Accordingly, this embodiment is designed so that a case in which a time spent on the movement of the center of gravity is 31 seconds or longer in 1 minute is determined as 1 minute of active state, and so that a case in which the time spent on the movement of the center of gravity is 30 seconds or less in 1 minute is determined as a non-active state (an end of the active state).

The advice reception part 327 receives, when a request for registration of a new piece of advice is issued from the advice provider terminal 50, the advice registration, and stores the new piece of advice in an advice information storage part 344 of the DB server 340.

The reservation reception part 328 is a function part for receiving reservation for remote rehabilitation when a request to reserve remote rehabilitation is issued from the wheelchair user terminal 50.

The DB server 340 is a server for storing various types of data, such as information about users, information about the sensors 14, information about alert, and information about advice, and is managed by a database management system (DBMS) (not shown) in an integrated manner.

As illustrated in FIG. 6, the DB server 340 in this embodiment includes, as main components, a user information storage part 341, the sensor information storage part 342, an alert information storage part 343, the advice information storage part 344, and a set condition storage part 345. The alert information storage part 343 is an area for storing information about alert generated by the AP server 320. The advice information storage part 344 is an area for storing information about advice.

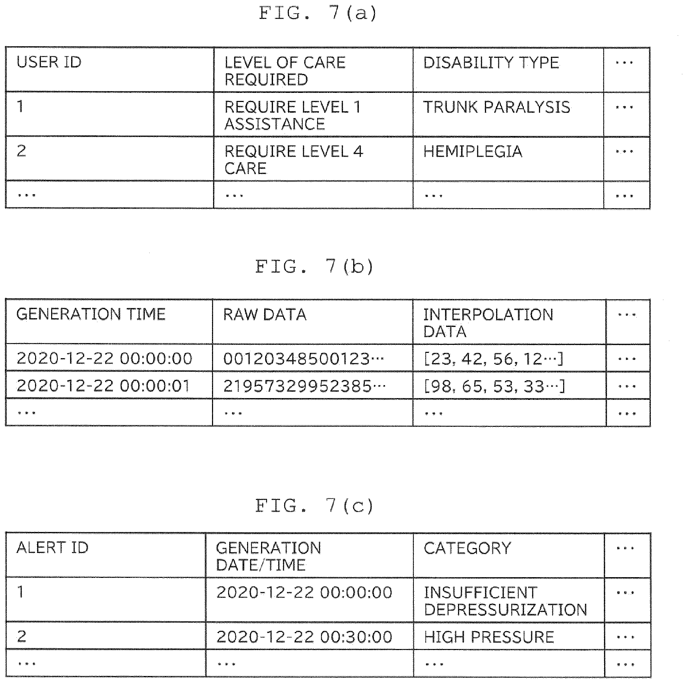

The user information storage part 341 is an area for storing information about users who use this pressure sore prevention system. A table such as the one shown in FIG. 7(a) is prepared in the user information storage part 341 to store such information as a "user ID," a "level of care required," and a "disability type."

The sensor information storage part 342 is an area for storing information that is acquired by the seating device 10 and transmitted to the management server 30, formatted data of this information, aggregated data obtained by aggregating the formatted data, and the like. A table such as the one shown in FIG. 7(b) is prepared in the sensor information storage part 342 to store such information as a "generation time," "raw data," and "interpolation data."

The alert information storage part 343 is an area for storing information about alert generated by the alert determination part 325. A table such as the one shown in FIG. 7(c) is prepared in the alert information storage part 343 to store such information as an "alert ID," a "generation date/time," and a "category."

The advice information storage part 344 is an area for storing information about a new piece of advice input from the advice provider terminal 50. A table (not shown) is prepared in the advice information storage part 344 to store such information as "advice contents," "advice creation date/time," and a "creator."

The set condition storage part 345 is an area for storing information about a set condition input from the wheelchair user terminal 50. A table (not shown) is prepared in the set condition storage part 345 to store, although not shown, such information as a "left ischial bone position," a "right ischial bone position," a "sacral bone position," a "bodily movement goal value," an "action detection reference value," and an "alert reference value."

The configuration of the management server 30 described above is an example, and the management server 30 may have other configurations as long as the desired object is attained.

### [User Terminal]

Next, the user terminal 50 in the pressure sore prevention system of this embodiment is described with reference to the drawings. Each user terminal 50 is information communication equipment used by a wheelchair user, a physical therapist, a care worker, a family member, a medical service worker, an insurance service person, or the like.

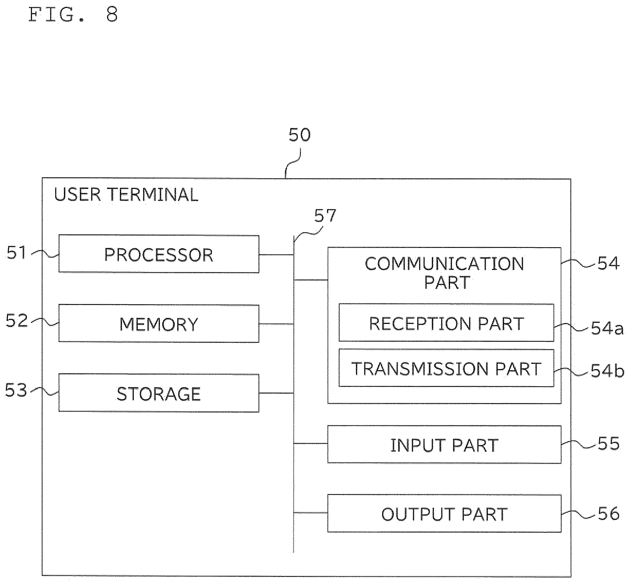

As illustrated in FIG. 8, the user terminal 50 in this embodiment includes, as main components, a processor 51, a memory 52, a storage 53, a communication part 54, an input part 55, and an output part 56. Those components are electrically connected to one another through a bus 57. For example, a PC, a smartphone, or a tablet terminal is usable as the user terminal 50.

The processor 51, the memory 52, the storage 53, the communication part 54, the input part 55, and the output part 56 are the same as the processor 31, the memory 32, the storage 33, the communication part 34, the input part 35, and the output part 36 described above. Description on portions common to the two sets of components are accordingly omitted.

Each user can use this pressure sore prevention system from a browser on his or her own user terminal 50. This pressure sore prevention system may be designed so as to be usable on a dedicated application installed in the user terminal 50.

The configuration of the user terminal 50 described above is an example, and the user terminal 50 may have other configurations as long as the desired object is attained.

### (Processing in Management Server)

Next, processing procedures executed on the management server 30 of the pressure sore prevention system of this embodiment are described. Programs (application programs) for causing the management server 30 to function as a server that executes the following processing procedures are stored on the management server 30 of the pressure sore prevention system of this embodiment, and the management server 30 executes one of the following processing procedures in response to a request from the user terminal 50.

### (Processing 1: Login Processing)

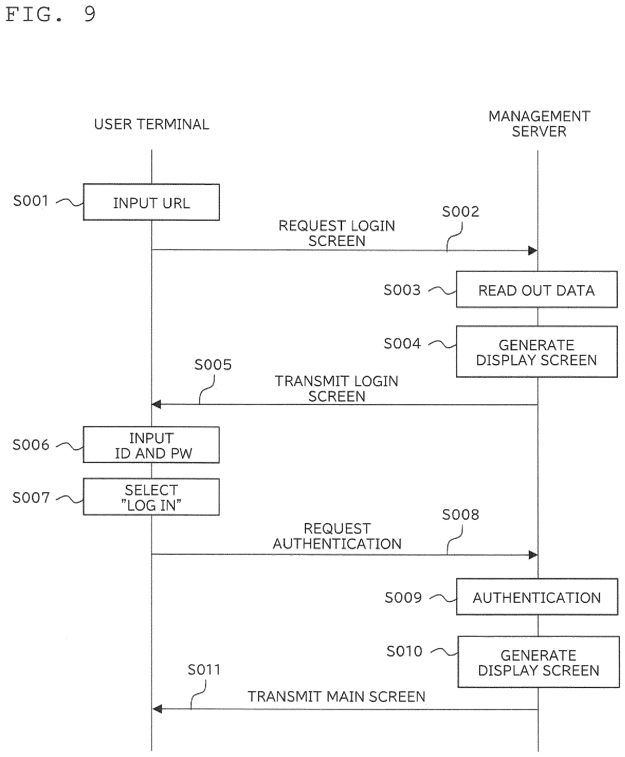

FIG. 9 is a sequence diagram of a case of logging in with the use of the user terminal 50. As illustrated in FIG. 9, when a URL is input on a browser of the user terminal 50 (Step S001), the user terminal 50 issues a request for a login screen 60 to the management server 30 (Step S002).

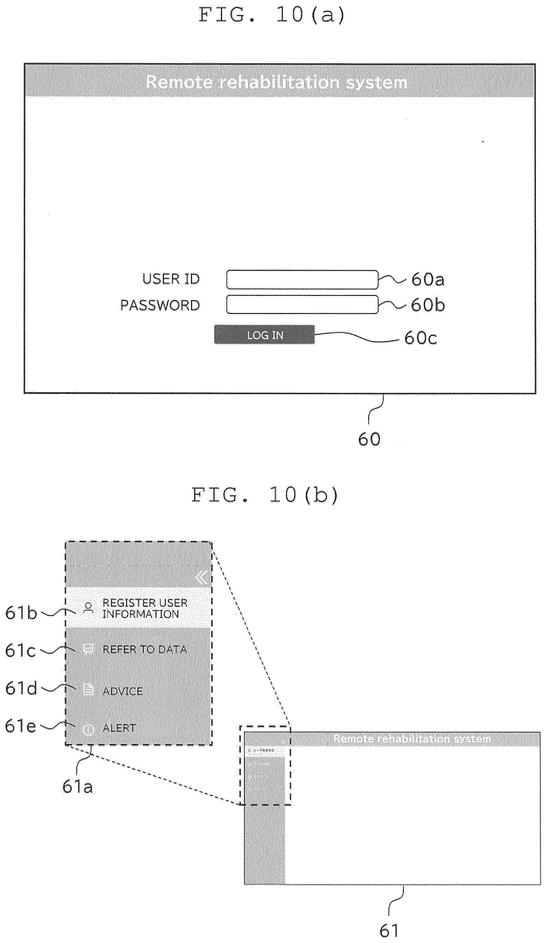

The management server 30 receives the request for the login screen 60, reads out data (Step S003), generates the login screen 60 (Step S004), and transmits the login screen 60 to the user terminal 50 (Step S005). The login screen 60 such as the one illustrated in FIG. 10(a) is displayed on the browser of the user terminal 50 having received the login screen 60.

When an ID and a password (PW) are input to an ID input field 60a and a password input field 60b, respectively, on the login screen 60 displayed on the browser of the user terminal 50 (Step S006), and a "login" button 60c displayed below the password input field 60b is selected (Step S007), the user terminal 50 issues a request for login authentication to the management server 30 (Step S008). The method of selection includes all selection methods, such as a click of a mouse, a press of an enter button of a keyboard, and a touch on a touch panel (the same is true in the following description).

Login methods other than input of an ID and a password on the login screen 60 may be employed. For example, a login method utilizing authentication by another SNS service, a login method cooperating with an outside system, and a login method utilizing biometric authentication, such as fingerprint authentication, face authentication, and iris authentication, are employable. In any case, a highly safe method is preferred in view of security.

The management server 30 receives the request for login authentication from the user terminal 50, executes authentication of the user (Step S009), generates a main screen 61 for the user who uses this user terminal 50 (Step S010), and transmits the main screen 61 to the user terminal 50 (Step S011). The browser of the user terminal 50 having received the main screen 61 displays the main screen 61 on which, as illustrated in FIG. 10(b), a main menu 61a is included in an upper left area. The main menu 61a illustrated in FIG. 10(b) includes a "register user information" button 61b, a "refer to data" button 61c, an "advice" button 61d, and an "alert" button 61e.

### (Processing 2: User Registration Processing)

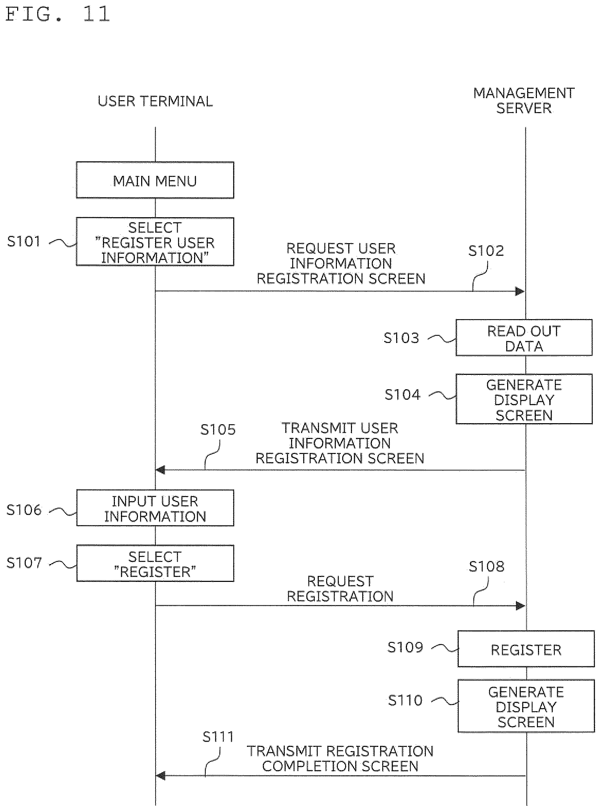

FIG. 11 is a sequence diagram of a case of user registration that uses the user terminal 50. As illustrated in FIG. 11, when the "register user information" button 61b of the main menu 61a (FIG. 10(b)) is selected on the browser of the user terminal 50 (Step S101), the user terminal 50 issues a request for a user information registration screen 62 to the management server 30 (Step S102).

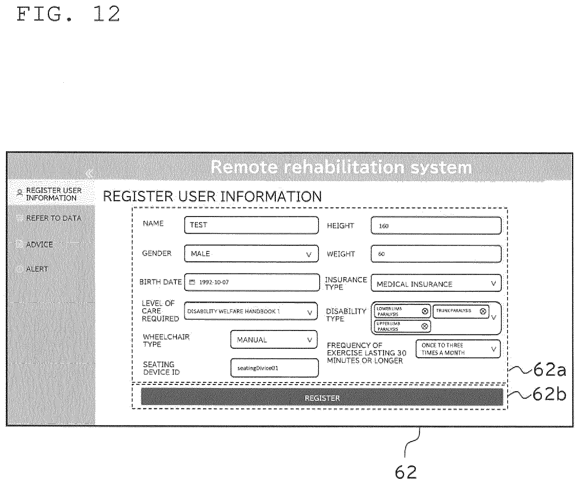

The management server 30 receives the request for the user information registration screen 62 from the user terminal 50, reads out data (Step S103), generates the user information registration screen 62 (Step S104), and transmits the user information registration screen 62 to the user terminal 50 (Step S105). The user information registration screen 62 such as the one illustrated in FIG. 12 is displayed on the browser of the user terminal 50 having received the user information registration screen 62.

The user information registration screen 62 illustrated in FIG. 12 includes a user information input portion 62a and a "register" button 62b. The user information input portion 62a includes input boxes for a "name," "gender," "birthdate," "height," the "level of care required," a "wheelchair type," a "seating device ID," a "weight," an "insurance type," the "disability type," a "frequency of exercise lasting 30 minutes or longer," and the like. The registration items given here are an example, and other items, for example, "age (age group)," "occupation," "place of living (address)," "walking capability/incapability," and a "disability site," may be included.

When user information is input in the user information input portion 62a of the user information registration screen 62 displayed on the browser of the user terminal 50 (Step S106), and the "register" button 62b is selected (Step S107), the user terminal 50 issues a request for user registration to the management server 30 (Step S108).

The management server 30 receives the request for user registration from the user terminal 50, stores (registers) the user information in the user information storage part 341 of the DB server 340 (Step S109), generates a registration completion screen (not shown) (Step S110), and transmits the registration completion screen to the user terminal 50 (Step S111).

### (Processing 3: Data Reference Processing)

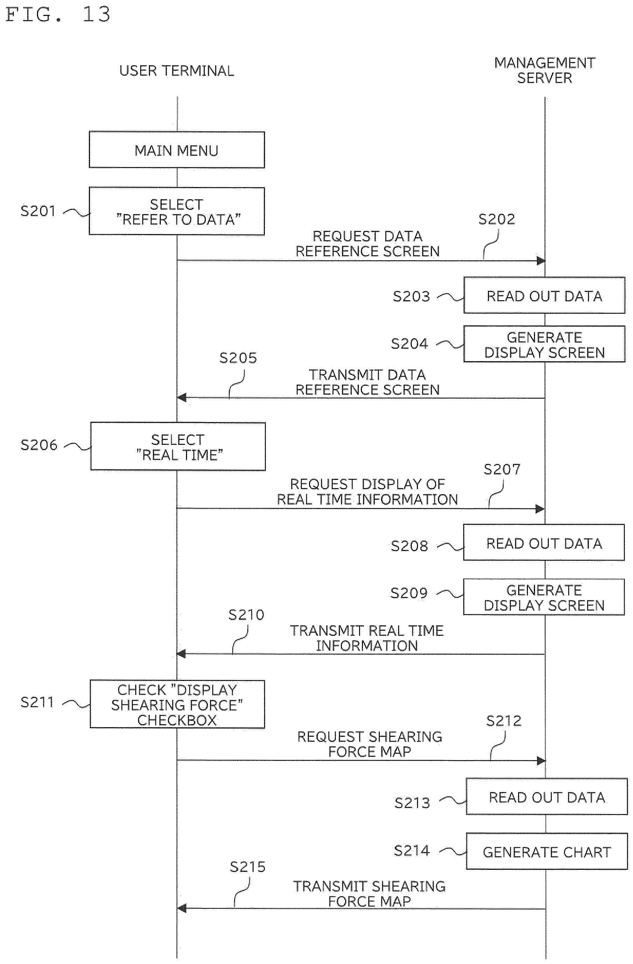

FIG. 13 is a sequence diagram of a case of referring to data with the use of the user terminal 50. As illustrated in FIG. 13, when the "refer to data" button 61c is selected from the main menu 61a (FIG. 10(b)) displayed on the user terminal 50 (Step S201), the user terminal 50 issues a request for the data reference screen 63 to the management server 30 (Step S202).

The management server 30 receives the request for the data reference screen 63 from the user terminal 50, reads out data (Step S203), generates the data reference screen 63 for the user (Step S204), and transmits the data reference screen 63 to the user terminal 50 (Step S205). The data reference screen 63 such as the one illustrated in FIG. 14(a) is displayed on the browser of the user terminal 50 having received the data reference screen 63.

As illustrated in FIG. 14(a), the data reference screen 63 in this embodiment is configured so that a "real time" mode or a "period specified" mode is selectable by switching between a "real time" tab 63a and a "period specified" tab 63b. In the "real time" mode, real time data can be referred to and, in the "period specified" mode, an average value of data in a period of the user's choice can be referred to.

As illustrated in FIG. 13, when the "real time" tab 63a is selected on the data reference screen 63 displayed on the browser of the user terminal 50 (Step S206), the user terminal 50 issues a request for display of real time information to the management server 30 (Step S207).

The management server 30 receives the request for real time information from the user terminal 50, reads out data (Step S208), generates real time information (Step S209), and transmits the real time information to the user terminal 50 (Step S210). The browser of the user terminal 50 having received the real time information displays a screen including the real time information, such as the one illustrated in FIG. 14(a).

The data reference screen 63 in the "real time" mode in this embodiment displays the seat surface heat map 81, which displays pressure applied to the seat surface X1 (the seat surface sensors 14a) in colors that vary depending on the magnitudes of pressure values, and the seat back heat map 82, which displays pressure applied to the back support X2 (the seat back sensors 14b) in colors that vary depending on the magnitudes of pressure values. In this embodiment, a point (center-of-gravity point) 81a indicating the position of the center of gravity is displayed on the seat surface heat map 81.

As illustrated in FIG. 14(a) and FIG. 14(b), in this embodiment, the checkbox 81b of "display shearing force" is displayed below the seat surface heat map 81. As illustrated in FIG. 13, when the checkbox 81b of "display shearing force" is checked on the browser of the user terminal 50 (Step S211), the user terminal 50 issues a request for display of the shearing force map 83 to the management server 30 (Step S212).

The management server 30 receives the request for the shearing force map 83 from the user terminal 50, reads out data (Step S213), generates the shearing force map 83 (Step S214), and transmits the shearing force map 83 to the user terminal 50 (Step S215). As illustrated in FIG. 14(b), the shearing force map 83 is displayed overlaid on the seat surface heat map 81 on the browser of the user terminal 50 having received the shearing force map 83. When the checkbox 81b of "display shearing force" is unchecked on the browser of the user terminal 50, the management server 30 executes switching so that the shearing force map 83 currently being displayed stops being displayed.

The seat surface heat map 81, the seat back heat map 82, and the shearing force map 83 that are displayed on the data reference screen 63 in the "real time" mode are generated based on data transmitted in real time to the management server 30.

As illustrated in FIG. 14(a), the data reference screen 63 in the "real time" mode in this embodiment displays, in addition to the seat surface heat map 81 and the seat back heat map 82, a "start recording" button 63c, an "end recording" button 63d, a "save" button 63e, an offset input portion 63f, a record list display portion 63g, a "play" button 63h, a "stop" button 63i, and others.

The "start recording" button 63c is a button for starting recording changes of the seat surface heat map 81 and the seat back heat map 82, and the "end recording" button 63d is a button for stopping the recording. When the "start recording button" 63c is selected on the browser of the user terminal 50, the management server 30 starts recording changes of the seat surface heat map 81 and the seat back heat map 82 and, when the "end recording" button 63d is selected, the management server 30 stops the recording.

The offset input portion 63f is a portion to which a numerical value for setting timing to start recording is input. A positive number, 0 (zero), or a negative number can be input to the offset input portion 63f. When a positive number (for example, "2") is input to the offset input portion 63f on the browser of the user terminal 50, the management server 30 starts recording at a point 2 seconds past the clicking of the "start recording" button 63c. When zero is input, the management server 30 starts recording at the same time as the clicking of the "start recording" button 63c. When a negative number (for example, "-2") is input, the management server 30 starts recording at a point 2 seconds prior to the clicking of the "start recording" button 63c.

The "save" button 63e is a button for saving the recorded changes of the seat surface heat map 81 and the seat back heat map 82. When the "save" button 63e is selected on the browser of the user terminal 50, the management server 30 saves the recording of changes of the seat surface heat map 81 and the seat back heat map 82.

The record list display portion 63g is a portion in which a list of data recorded in the past is displayed. The record list 94 displayed in the record list display portion 63g includes a "start date/time," an "end date/time," and an "offset."

The "play" button 63h is a button for playing the recorded heat maps 81 and 82 that are selected from the record list 94, and the "stop" button 63i is a button for stopping the playing of the heat maps 81 and 82.

When a record of the user's choice is selected from the record list 94 and the "play" button 63h is selected on the browser of the user terminal 50, the heat maps 81 and 82 of this record are played. When the "stop" button 63i is clicked during the playing of the heat maps 81 and 82, the playing of the heat maps 81 and 82 is stopped.

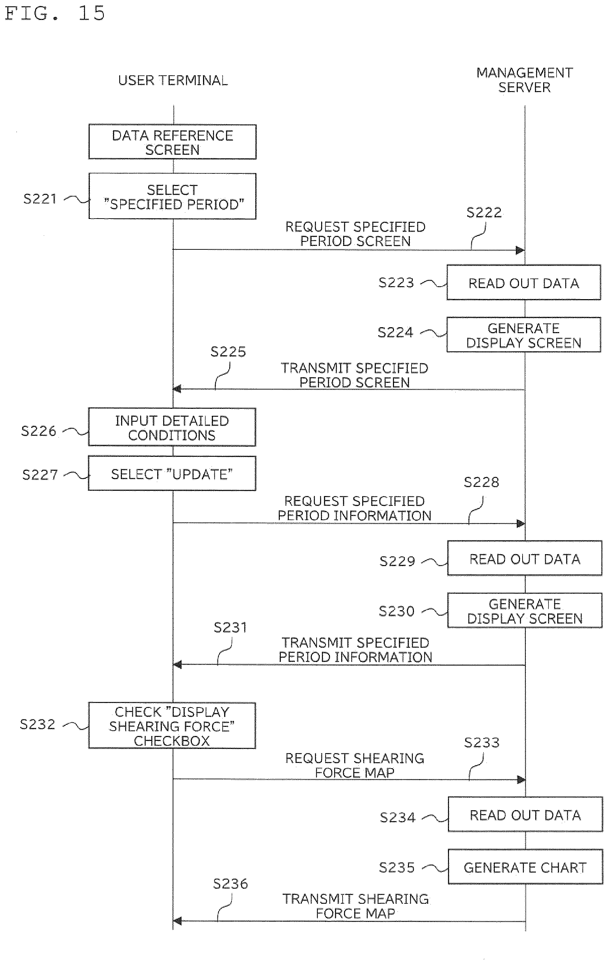

As illustrated in FIG. 15, when the "specified period" tab is selected on the data reference screen 63 displayed on the browser of the user terminal 50 (Step S221), the user terminal 50 issues a request for display of data in a specified period to the management server 30 (Step S222). The management server 30 receives the request for a specified period screen, reads out the data (Step S223), generates the specified period screen (Step S224), and transmits the specified period screen to the user terminal 50 (Step S225).

As illustrated in FIG. 16(a), the data reference screen 63 in the "specified period" mode includes a simple specification portion 63j for specifying a period in a simplified manner, a details specification portion 63k for specifying a period in detail, and an "update" button 63m. In the simple specification portion 63j, "today," "yesterday," "this month," "past 1 week," and "past 1 month" buttons are prepared so that a period can be specified by clicking any of the buttons.

The details specification portion 63k includes a year-month-day input portion and a time input portion. The year-month-day input portion is boxes to each of which a year, a month, and a day are input, and the time input portion is boxes to each of which a time is input. The "update" button 63m is a button for updating information.

When information is input to the year-month-day input portion and the time input portion (Step S226) and the "update" button 63m is selected (Step S227) on the browser of the user terminal 50, the user terminal 50 issues a request for display of specified period information to the management server 30 (Step S228).

The management server 30 receives the request for the specified period information from the user terminal 50, reads out data (Step S229), generates the specified period information (Step S230), and transmits the specified period information to the user terminal 50 (Step S231). A screen including the specified period information such as the one illustrated in FIG. 16(a) is displayed on the browser of the user terminal 50 having received the specified period information.

The specified period information in this embodiment includes the bodily movement item list 91, the seat surface heat map 81, the seat back heat map 82, the shearing force map 83, the shearing force graph 84, and the seated body pressure graph 85.

The bodily movement item list 91 displays, for each of bodily movement items, a list including the number of times of the bodily movement today, an average number of times of the bodily movement averaged over a period, and a goal value of the number of times of the bodily movement. The bodily movement items include, among others, a "wheelchair seated time," the "number of times of taking a seat on the wheelchair," "push-ups (long, short)," "weight shift left (long, short)," "weight shift right (long, short), and "active."

The seat surface heat map 81 displays an average seated body pressure of a specified period in colors that vary depending on the magnitudes of pressure values. The seat back heat map 82 displays an average back pressure of the specified period in colors that vary depending on the magnitudes of pressure values. The same center-of-gravity point 81a as the one on the seat surface heat map 81 that is displayed on the data reference screen 63 in the "real time" mode is displayed on the seat surface heat map 81.

The checkbox 81b of "display shearing force" is displayed below the seat surface heat map 81. As illustrated in FIG. 15, when the checkbox 81b of "display shearing force" is checked on the browser of the user terminal 50 (Step S232), the management server 30 issues a request for display of the shearing force map 83 to the management server 30 (Step S233).

The management server 30 receives the request for the shearing force map 83 from the user terminal 50, reads out data (Step S234), generates the shearing force map 83 (Step S235), and transmits the shearing force map 83 to the user terminal 50 (Step S236). As illustrated in FIG. 14(b), the shearing force map 83 is displayed overlaid on the seat surface heat map 81 on the browser of the user terminal 50 having received the shearing force map 83.

When the checkbox 81b of "display shearing force" is unchecked on the browser of the user terminal 50, the management server 30 executes switching so that the shearing force map 83 currently being displayed stops being displayed.

The shearing force graph 84 displays changes in shearing force in the right ischial bone, the left ischial bone, and the sacral bone within a specified period. The seated body pressure graph 85 displays changes in seated body pressure in the right ischial bone, the left ischial bone, and the sacral bone within the specified period.

The seat surface heat map 81, the seat back heat map 82, and the shearing force map 83 that are displayed on the data reference screen 63 in the "specified period" mode are generated based on an average value of the seated body pressure averaged over a specified period, and the like.

A "settings" button 63n (FIG. 16(a)) displayed in an upper right area of the data reference screen 63 in the "specified period" mode is a button for moving to a screen for setting a goal value of an action, and setting the right ischial bone, the left ischial bone, and the sacral bone to be measured. Processing executed when the "settings" button 63n is selected is described later.

### (Processing 4: Action Inquiry Processing)

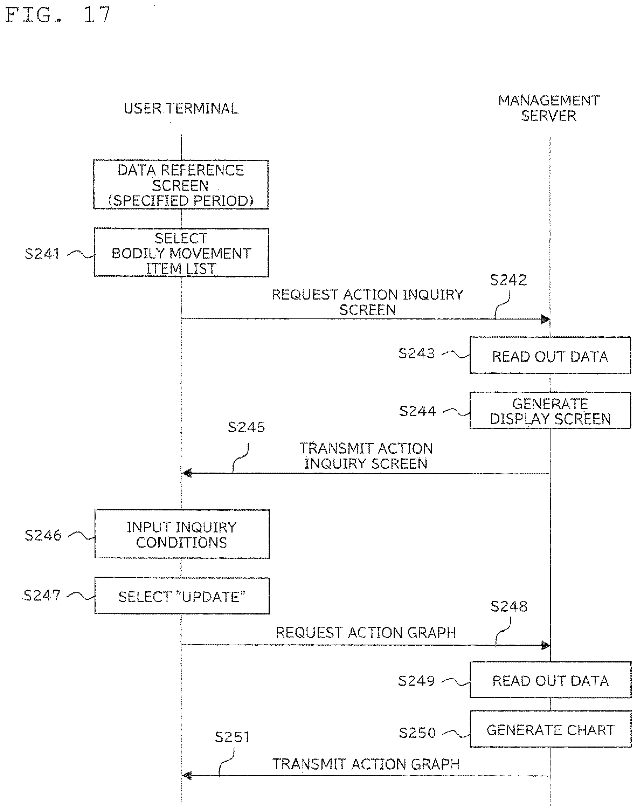

FIG. 17 is a sequence diagram of a case of executing action inquiry with the use of the user terminal 50. As illustrated in FIG. 17, when any action is selected from the bodily movement item list 91 of the data reference screen 63 in the "specified period" mode on the browser of the user terminal 50 (Step S241), the user terminal 50 issues a request for an action inquiry screen 64 to the management server 30 (Step S242).

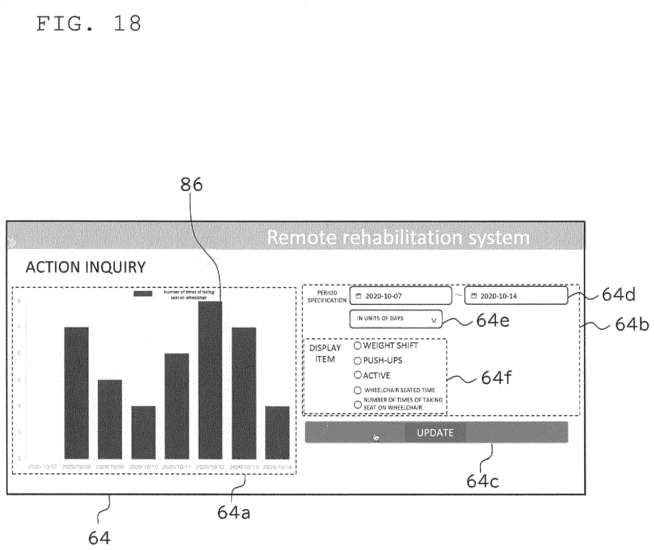

The management server 30 receives the request for the action inquiry screen 64 from the user terminal 50, reads out data (Step S243), generates the action inquiry screen 64 about the selected action (Step S244), and transmits the action inquiry screen 64 to the user terminal 50 (Step S245). The action inquiry screen 64 such as the one illustrated in FIG. 18 is displayed on the browser of the user terminal 50 having received the action inquiry screen 64.

The action inquiry screen 64 in this embodiment includes an action graph display portion 64a, an inquiry condition input portion 64b, and an "update" button 64c. The inquiry condition input portion 64b includes a period specification portion 64d, a unit selection portion 64e, and a display item selection portion 64f.

The action graph display portion 64a is a portion in which the action graph 86 about a selected action is displayed. The period specification portion 64d is a portion for inputting a period that is a target of action inquiry. The unit selection portion 64e is a portion for selecting from hourly-basis action inquiry and daily-basis action inquiry. The display item selection portion 64f is a portion for selecting an action to be referred to. The "update" button 64c is a button for displaying the action graph 86 that matches determined display conditions.

As illustrated in FIG. 17, when inquiry conditions are input to the inquiry condition input portion 64b displayed on the browser of the user terminal 50 (Step S246), and the "update" button 64c is selected (Step S247), the user terminal 50 issues a request for the action graph 86 that matches the inquiry conditions to the management server 30 (Step S248).

The management server 30 receives the request for the action graph 86 from the user terminal 50, reads out data (Step S249), generates the action graph 86 (Step S250), and transmits the action graph 86 to the user terminal 50 (Step S251). The action graph 86 such as the one illustrated in FIG. 18 is displayed on the browser of the user terminal 50 having received the action graph 86. The action graph 86 shown in FIG. 18 indicates the number of times of taking a seat on the wheelchair, with the axis of ordinate indicating the number of times and the axis of abscissa indicating a time. The axis of ordinate and the axis of abscissa of the action graph 86 vary depending on set inquiry conditions.

### (Processing 5: Setting Processing)

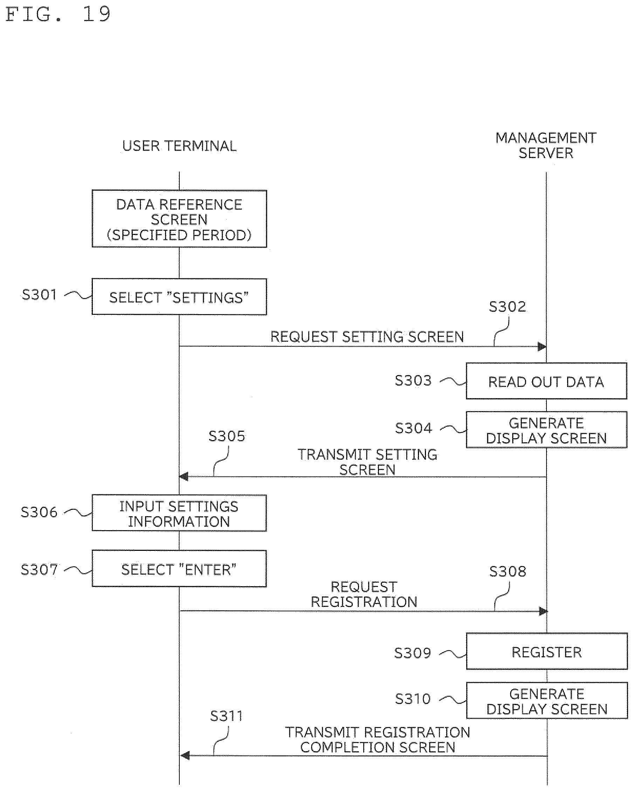

FIG. 19 is a sequence diagram of a case of setting positions of the ischial bones and the sacral bone to be measured, a goal value of each action, a reference value for detection of an action, and an alert reference value with the use of the user terminal 50. As illustrated in FIG. 19, when the "settings" button 63n displayed in an upper right area of the data reference screen 63 of FIG. 16(a) in the "period specified" mode is selected on the browser of the user terminal 50 (Step S301), the user terminal 50 issues a request for a setting screen 65 to the management server 30 (Step S302).

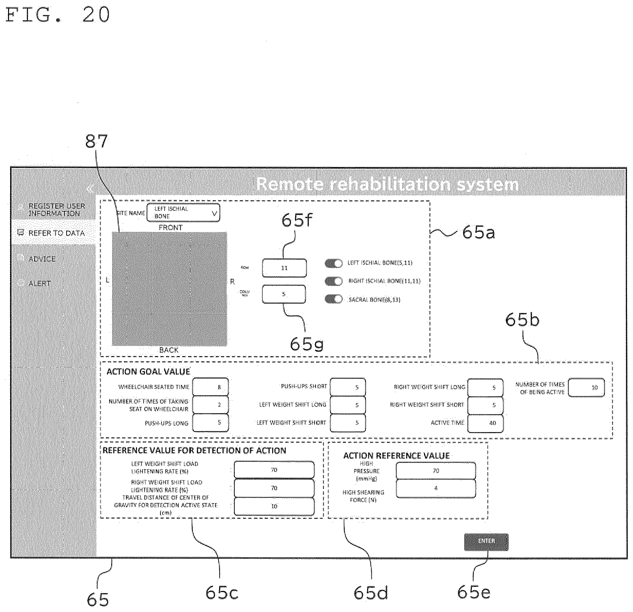

The management server 30 receives the request for the setting screen 65 from the user terminal 50, reads out data (Step S303), generates the setting screen 65 for the user (Step S304), and transmits the setting screen 65 to the user terminal 50 (Step S305). The setting screen 65 such as the one illustrated in FIG. 20 is displayed on the browser of the user terminal 50 having received the setting screen 65.

The setting screen 65 in this embodiment includes a site setting portion 65a, an action goal setting portion 65b, an action detection reference setting portion 65c, an alert reference setting portion 65d, and an "enter" button 65e.

The site setting portion 65a is a portion for determining a site to be set. In this embodiment, a site is selectable from three options that are the left ischial bone, the right ischial bone, and the sacral bone in the site setting portion 65a. An axis of ordinate and an axis of abscissa are displayed in a setting map 87 displayed in the site setting portion 65a, and a measurement site can be set by clicking on any point on the map. A row display block 65f and a column display block 65g displayed to the right of the setting map 87 display coordinates of the set measurement site in numerical values.

The action goal setting portion 65b is a portion for setting goal values of the "wheelchair seated time," the "number of times of taking a seat on the wheelchair," "push-ups (long, short)," "weight shift left (long, short)," "weight shift right (long, short), an "active time," and the "number of times of being active." This embodiment is designed so that the length of time of an action and the number of times of the action per hour are calculated for each action when the "wheelchair seated time" is input along with a goal length of time and a goal number of times of the action.

The action detection reference setting portion 65c is a portion for setting a load lightening rate of left weight shift and right weight shift, and setting a center-of-gravity moving distance for detecting the active state. Left weight shift load lightening means a rate of load lightening observed on the right seat surface when the weight is shifted to the left. The right weight shift load lightening means a rate of load lightening observed on the left seat surface when the weight is shifted to the right.

The alert reference setting portion 65d is a portion for setting a reference value for issuing alert. In this embodiment, reference values can be set with respect to high pressure (mmHg) and high shearing force (N). The "enter" button 65e is a button for establishing set conditions input for the respective items.

The issuing of alert may be triggered by an item other than high pressure and high shearing force. For example, alert may be issued when the number of times of push-ups or the number of times of weight shift falls short of the action goal value, or when the number of times of taking a seat on the wheelchair falls short of the action goal value.

Other than trouble in the form of high pressure, high shearing force, and a failure to reach the action goal value, a symptom, a disability, the level of care required, a day-to-day physical condition, or a similar factor may be set as a trigger for issuing alert. For example, a typical reference value may be set on the management server 30 to be set, in advance, as a reference value for issuing alert.

The typical reference value set by the management server 30 may be set based on, for example, the "level of care required" and the "disability type" (FIG. 7(a)) stored in the user information storage part 341 (FIG. 6). To give an example, when the "level of care required" is high or the disability type is paralysis of other body parts on top of a leg disability, changing body posture may be difficult in the first place, and the use of a normal reference value may accordingly result in a situation in which alert is continuously issued.

In order to avoid this situation, for some of settings items, the management server 30 may set typical reference values based on the "level of care required" and the "disability type" of the user information storage part 341 so that alert is not issued on every count (including stopping issuing of alert altogether).

As illustrated in FIG. 19, when the pieces of settings information are input (Step S306) and the "enter" button 65e is selected (Step S307) on the browser of the user terminal 50, the user terminal 50 issues a request for registration of the settings information to the management server 30 (Step S308).

The management server 30 receives the request for registration of the settings information from the user terminal 50, stores (registers) the settings information in the set condition storage part 345 (FIG. 6) of the database server 340 (Step S309), generates a registration completion screen (not shown) (Step S310), and transmits the generated registration completion screen (not shown) to the user terminal 50 (Step S311).

### (Processing 6: Advice List Reference Processing)

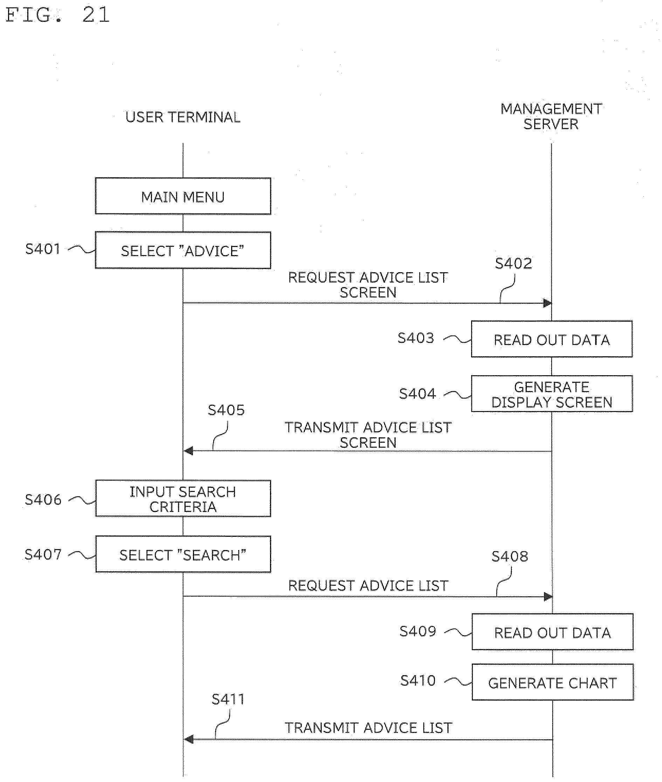

FIG. 21 is a sequence diagram of a case of referring to the advice list 92 with the use of the user terminal 50. As illustrated in FIG. 21, when the "advice" button 61d of the main menu 61a (FIG. 10(b)) displayed on the browser of the user terminal 50 is selected (Step S401), the user terminal 50 issues a request for the advice list screen 66 to the management server 30 (Step S402).

The management server 30 receives the request for the advice list screen 66 from the user terminal 50, reads out data (Step S403), generates the advice list screen 66 (Step S404), and transmits the advice list screen 66 to the user terminal 50 (Step S405). The advice list screen 66 such as the one illustrated in FIG. 22 is displayed on the browser of the user terminal 50 having received the advice list screen 66.

The advice list screen 66 in this embodiment includes a search criteria input portion 66a to which search criteria are input, a "new advice" button 66b for newly creating advice, an advice list display portion 66c for displaying the advice list 92 that matches the search criteria, and a "search" button 66d.

The search criteria input portion 66a includes setting fields that are a "No. field," a "category field," a "date field," a "flag field," and an "alert field." The advice list 92 displayed in the advice list display portion 66c includes the "inquiry field," the "No. field," the "category field," the "date field," the "flag field," the "advice field," the "alert No./category field," the "alert details field," and the "delete field."

When one of the search criteria out of "No.," "category," "date," "flag," and "alert" is set in the search criteria input portion 66a (Step S406) and the "search" button 66d is selected (Step S407) on the browser of the user terminal 50, the user terminal 50 issues a request for the advice list 92 to the management server 30 (Step S408).

The management server 30 receives the request for the advice list 92 from the user terminal 50, reads out data (Step S409), generates the advice list 92 that matches the search criteria (Step S410), and transmits the advice list 92 to the user terminal 50 (Step S411). The advice list 92 such as the one in FIG. 22 is displayed in the advice list display portion 66c on the browser of the user terminal 50 having received the advice list 92.

### (Processing 7: Advice Inquiry Processing)

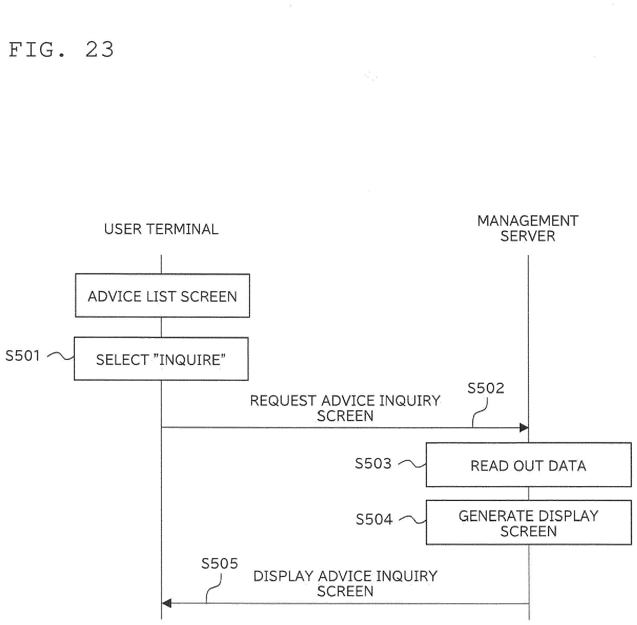

FIG. 23 is a sequence diagram of a case of issuing an inquiry about a specific piece of advice with the use of the user terminal 50. As illustrated in FIG. 23, when any "inquire" button is selected (Step S501) on the advice list 92 displayed on the browser of the user terminal 50, the user terminal 50 issues a request for display of the advice inquiry screen 67 that indicates details of the specified piece of advice to the management server 30 (Step S502).

The management server 30 receives the request for the advice inquiry screen 67 from the user terminal 50, reads out data (Step S503), generates the advice inquiry screen 67 (Step S504), and transmits the advice inquiry screen 67 to the user terminal 50 (Step S505). The advice inquiry screen 67 such as the one illustrated in FIG. 24 is displayed on the browser of the user terminal 50.

This advice inquiry screen 67 includes a basic information display portion 67a, the bodily movement item list 91, the seat surface heat map 81, the seat back heat map 82, the shearing force map 83 (see FIG. 14(a) and FIG. 14(b)), the shearing force graph 84, and the seated body pressure graph 85. The basic information display portion 67a includes a "No. field," a "period specification field," a "category field," a "flag field," an "alert field," and an "advice field."

### (Processing 8: Alert Inquiry Processing 1)

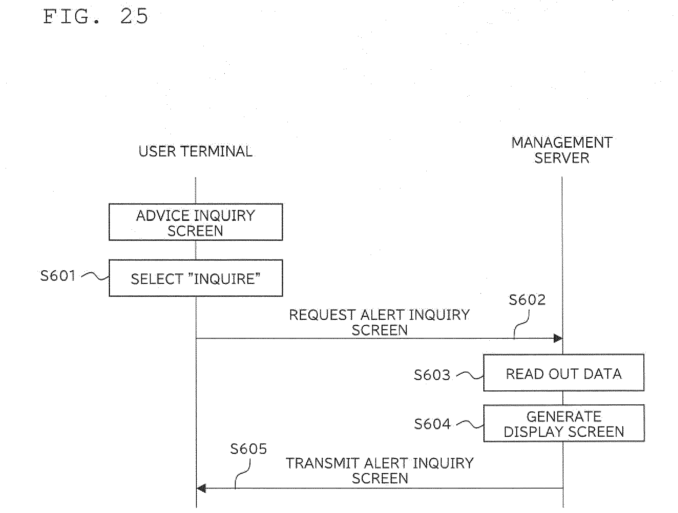

FIG. 25 is a sequence diagram of a case of issuing an inquiry about details of a specific piece of alert from the advice inquiry screen 67 displayed on the browser of the user terminal 50. As illustrated in FIG. 25, when an "inquire" button by the alert field is selected on the browser of the user terminal 50 (Step S601), the user terminal 50 issues a request for the alert inquiry screen 70 that indicates details of the specified piece of alert to the management server 30 (Step S602).

The management server 30 receives the request for the alert inquiry screen 70 from the user terminal 50, reads out data (Step S603), generates the alert inquiry screen 70 (Step S604), and transmits the alert inquiry screen 70 to the user terminal 50 (Step S605). The alert inquiry screen 70 such as the one illustrated in FIG. 31(b) is displayed on the browser of the user terminal 50 having received the alert inquiry screen 70. Details of the alert inquiry screen 70 are described later.

### (Processing 9: Alert Inquiry Processing 2)

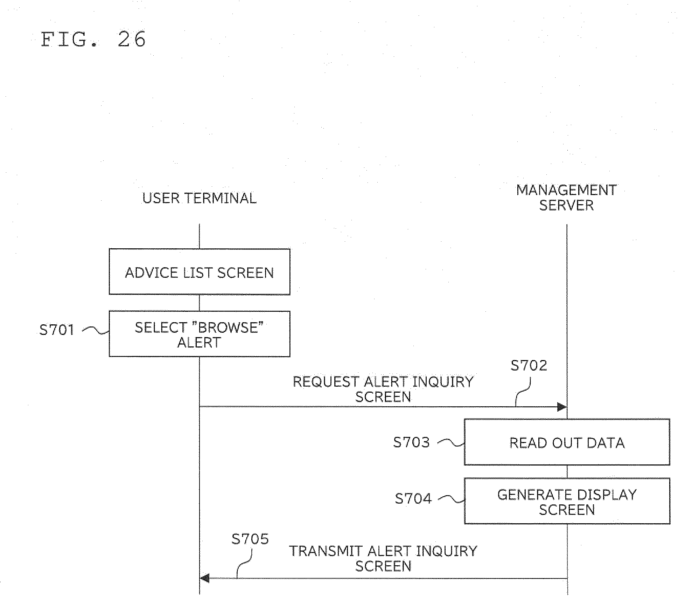

FIG. 26 is a sequence diagram of a case of issuing an inquiry about details of a specific piece of alert from the advice list screen 66 displayed on the browser of the user terminal 50. This processing is the same as the Processing 8 described above. Specifically, as illustrated in FIG. 26, when any "browse" button is selected from alert details of the advice list 92 on the browser of the user terminal 50 (Step S701), the user terminal 50 issues a request for the alert inquiry screen 70 about the specified piece of alert to the management server 30 (Step S702).

The management server 30 receives the request for the alert inquiry screen 70 from the user terminal 50, reads out data (Step S703), generates the alert inquiry screen 70 (Step S704), and transmits the alert inquiry screen 70 to the user terminal 50 (Step S705). The alert inquiry screen 70 such as the one illustrated in FIG. 31(b) is displayed on the browser of the user terminal 50 having received the alert inquiry screen 70. Details of the alert inquiry screen 70 are described later.

### (Processing 10: Advice Deletion Processing)

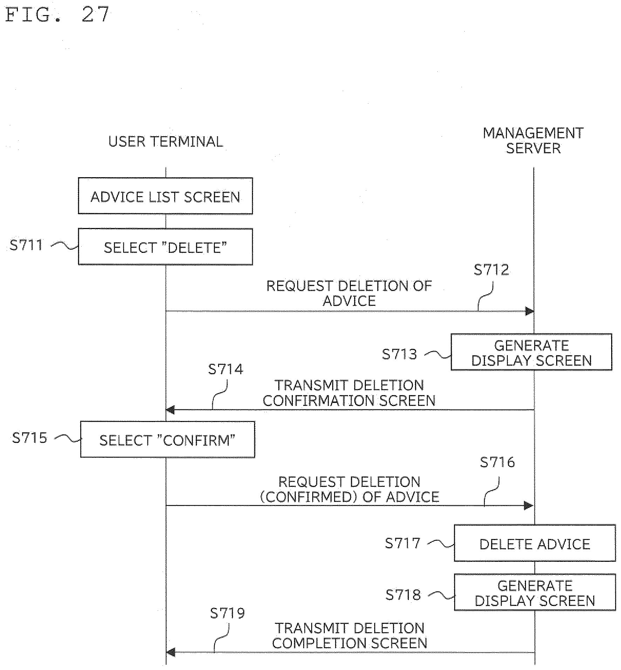

FIG. 27 is a sequence diagram of a case of deleting a specific piece of advice from the advice list screen 66 displayed on the user terminal 50. As illustrated in FIG. 27, when any "delete" button is selected from the delete field of the advice list 92 displayed on the browser of the user terminal 50 (Step S711), the user terminal 50 issues a request for deletion of the specified piece of advice to the management server 30 (Step S712).

The management server 30 receives the request for deletion of the advice from the user terminal 50, generates a deletion confirmation screen (not shown) to be displayed as a pop-up window (Step S713), and transmits the deletion confirmation screen to the user terminal 50 (Step S714).

When a "confirm" button is selected on the deletion confirmation screen displayed as the pop-up window on the browser of the user terminal 50 (Step S715), the user terminal 50 issues a request for deletion (confirmed) of the piece of advice to the management server 30 (Step S716).

The management server 30 receives the request for deletion (confirmed) of the advice from the user terminal 50, and deletes the piece of advice from the advice information storage part 344 of the DB server 340 (Step S717), generates a deletion completion screen (not shown) (Step S718), and transmits the generated deletion completion screen to the user terminal 50 (Step S719).

### (Processing 11: New Advice Registration Processing)

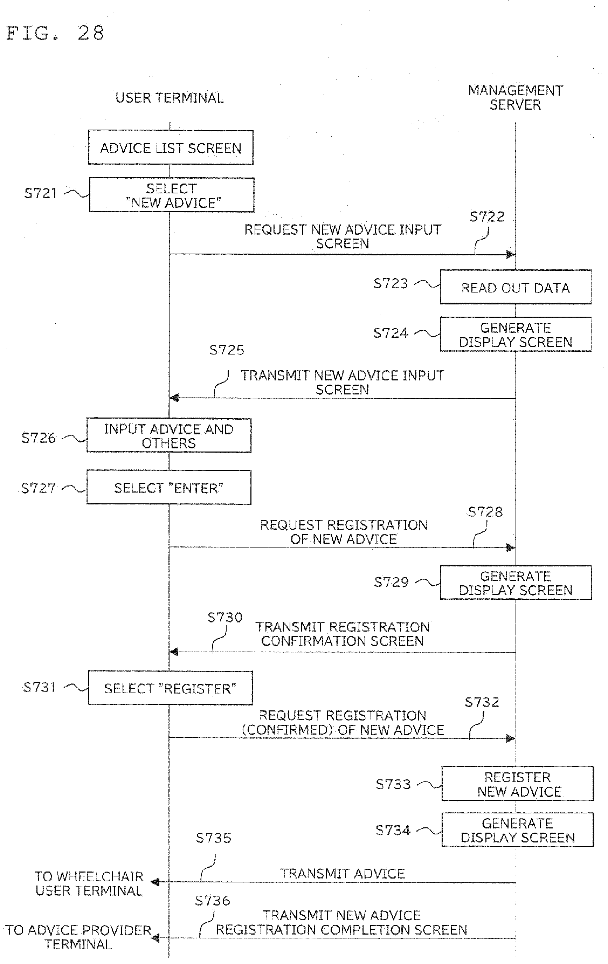

FIG. 28 is a sequence diagram of a case in which a physical therapist newly registers advice through the user terminal 50. As illustrated in FIG. 28, when the "new advice" button is selected on the advice list screen 66 displayed on the browser of the user terminal 50 (Step S721), the user terminal 50 issues a request for the new advice input screen 68 to the management server 30 (Step S722).

The management server 30 receives the request for the new advice input screen 68 from the user terminal 50, reads out data (Step S723), generates the new advice input screen 68 (Step S724), and transmits the new advice input screen 68 to the user terminal 50 (Step S725). The new advice input screen 68 such as the one illustrated in FIG. 29(a) is displayed on the browser of the user terminal 50 having received the new advice input screen 68.

This new advice input screen 68 includes a condition setting portion 68a, an advice input portion 68b, and an "enter" button 68c, as well as a list display portion 68d, the seat surface heat map 81, the seat back heat map 82, the shearing force map 83 (not shown), the shearing force graph 84, and the seated body pressure graph 85. The condition setting portion 68a includes a "period specification field," a "category field," a "flag field," and an "alert field."

When conditions and advice are input on the new advice input screen 68 displayed on the browser of the user terminal 50 (Step S726) and the "enter" button 68c is selected (Step S727), the user terminal 50 issues a request for registration of new advice to the management server 30 (Step S728).

The management server 30 receives the request for registration of new advice from the user terminal 50, generates a registration confirmation screen 68e to be displayed as a pop-up window as illustrated in FIG. 29(b) (Step S729), and transmits the registration confirmation screen 68e to the user terminal 50 (Step S730). The registration confirmation window 68e such as the one illustrated in FIG. 29(b) is displayed as a pop-up window on the browser of the user terminal 50.

When a "register" button 68f is selected on the registration confirmation screen 68e displayed as the pop-up window on the browser of the user terminal 50 (Step S731), the user terminal 50 issues a request for registration (confirmed) of the new advice to the management server 30 (Step S732).

The management server 30 receives the request for registration (confirmed) of the new advice from the user terminal 50, registers the piece of advice in the advice information storage part 344 of the DB server 340 (Step S733), transmits the piece of advice to the wheelchair user terminal 50 (Step S735), and generates (Step S734) and transmits (Step S736) a registration completion screen (not shown) to the advice provider terminal 50. As is the case for alert, advice is transmitted to an SNS installed in the user terminal 50 (viewable on the user terminal 50) in this embodiment. This increases the chance of advice being noticed by the wheelchair user, and enables quick response (action). However, advice may be transmitted via an electronic mail viewable on the user terminal 50.

### (Processing 12: Alert List Reference Processing)

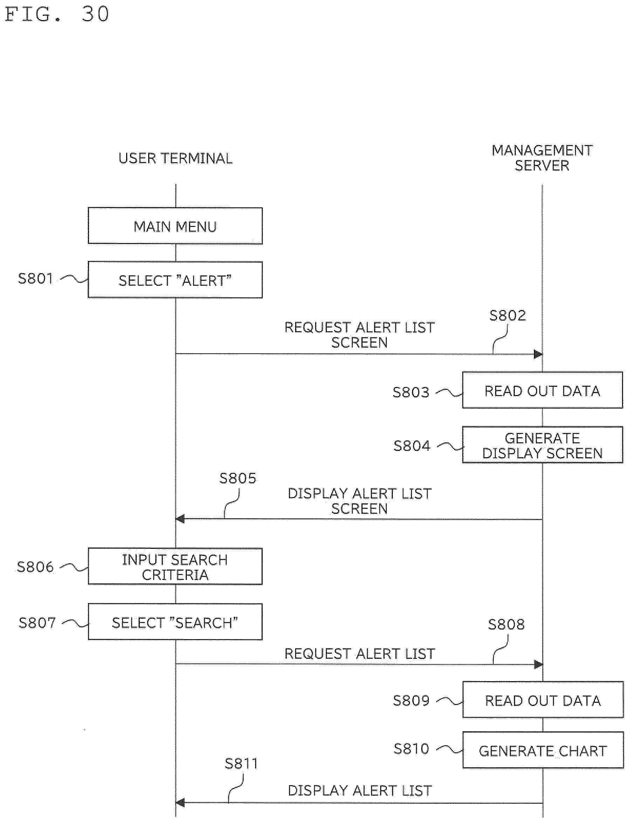

FIG. 30 is a sequence diagram of a case of referring to the alert list 93 from the main screen 61 displayed on the user terminal 50. As illustrated in FIG. 30, when the "alert" button 61e of the main menu 61a (FIG. 10(b)) is selected on the browser of the user terminal 50 (Step S801), the user terminal 50 issues a request for the alert list screen 69 to the management server 30 (Step S802).

The management server 30 receives the request for the alert list screen 69 from the user terminal 50, reads out data (Step S803), generates the alert list screen 69 (Step S804), and transmits the alert list screen 69 to the user terminal 50 (Step S805). The alert list screen 69 such as the one illustrated in FIG. 31(a) is displayed on the browser of the user terminal 50 having received the alert list screen 69.

The alert list screen 69 in this embodiment includes a search criteria input portion 69a to which search criteria are input, an alert list display portion 69b for displaying the alert list 93 that matches the search criteria, and a "search" button 69c. The search criteria input portion 69a includes setting fields that are a "No. field," a "category field," a "date field," and a "flag field." The alert list 93 displayed in the advice list display portion 69b includes the "inquiry field," the "No. field," the "category field," the "date field," the "flag field," an "alert contents field," and a "related advice field."

When a criterion is set in one of the "No. field," the "category field," the "date field," the "flag field," and the "alert field" of the search criteria input portion 69a (Step S806), and the "search" button 69c is selected (Step S807) on the alert list screen 69 displayed on the browser of the user terminal 50, the user terminal 50 issues a request for the alert list 93 to the management server 30 (Step S808).

The management server 30 receives the request for the alert list 93 from the user terminal 50, reads out data (Step S809), generates the alert list 93 that matches the search criterion (Step S810), and transmits the alert list 93 to the user terminal 50 (Step S811). The alert list 93 such as the one in FIG. 31(a) is displayed in the alert list display portion 69b on the browser of the user terminal 50 having received the alert list 93.

### (Processing 13: Alert Inquiry Processing 3)

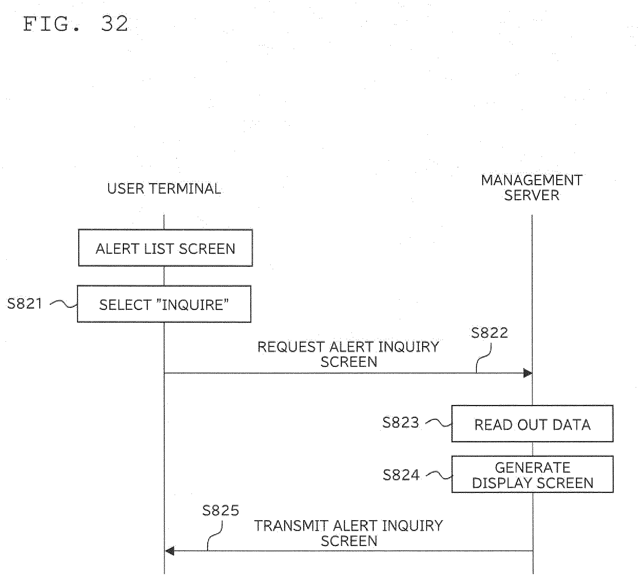

FIG. 32 is a sequence diagram of a case of issuing an inquiry about details of a specific piece of alert from the alert list screen 69 displayed on the user terminal 50. As illustrated in FIG. 32, when any "inquire" button is selected from the alert list 93 on the browser of the user terminal 50 (Step S821), the user terminal 50 issues a request for the alert inquiry screen 70 about the specified piece of alert to the management server 30 (Step S822).

The management server 30 receives the request for the alert inquiry screen 70 from the user terminal 50, reads out data (Step S823), generates the alert inquiry screen 70 (Step S824), and transmits the alert inquiry screen 70 to the user terminal 50 (Step S825). The alert inquiry screen 70 such as the one illustrated in FIG. 31(b) is displayed on the browser of the user terminal 50 having received the alert inquiry screen 70.

The alert inquiry screen 70 in this embodiment includes a basic information display portion 70a, an "input advice" button 70b, a "return" button 70c, a list display portion 70d, the seat surface heat map 81, the seat back heat map 82, the shearing force map 83 (not shown), the shearing force graph 84, and the seated body pressure graph 85. The basic information display portion 70a includes a "No. field," an "issued period field," a "category field," a "flag field," an "alert field," an "advice field," and an "inquire" button. The user of the user terminal 50 can view contents of alert and various types of data (a list, a map, a graph, and the like) associated with the alert from the alert inquiry screen 70.

### (Processing 14: Related Advice Inquiry Processing)

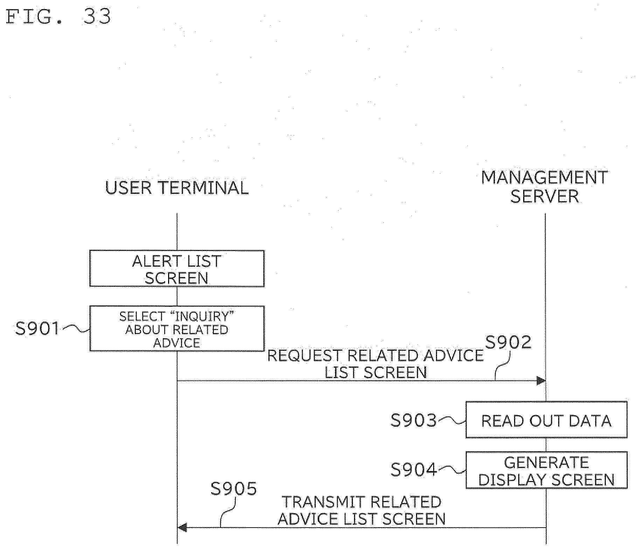

FIG. 33 is a sequence diagram of a case of referring to, from the alert inquiry screen 70 displayed on the user terminal 50, advice related to a piece of alert of the alert inquiry screen 70. As illustrated in FIG. 33, when the "inquire" button displayed to the right of the advice field is selected (Step S901) on the alert inquiry screen 70 (FIG. 31(b)) displayed on the browser of the user terminal 50, the user terminal 50 issues a request for a related advice list (not shown) related to the displayed piece of alert to the management server 30 (Step S902).

The management server 30 receives the request for the related advice list from the user terminal 50, reads out data (Step S903), generates the related advice list (Step S904), and transmits the related advice list to the user terminal 50 (Step S905). The related advice list similar to the advice list 92 of FIG. 22 is displayed on the browser of the user terminal 50 having received the related advice list.

According to the pressure sore prevention system of this embodiment, the wheelchair user can receive alert and advice in real time and, by changing the body posture each time alert or advice is received, can reduce the risk of developing a pressure sore. In addition, the wheelchair user can check his her own sitting posture type, posture misalignment, and the like with charts easy to understand visually, such as the seat surface heat map 81 and the seat back heat map 82, and the shearing force map 83, the shearing force graph 84, the seated body pressure graph 85, and the action graph 86 as well.

According to the pressure sore prevention system of this embodiment, not only a wheelchair user but also a physical therapist, a care worker, a family member, a medical service worker, an insurance service person, or the like can use his or her own user terminal 50 to view information provided from the management server 30 and share information among involved persons including the wheelchair user, and can accordingly provide appropriate support based on shared awareness to the wheelchair user.

### (Another Embodiment)

In the embodiment described above, application to a pressure sore prevention system for preventing a pressure sore of a wheelchair user is taken as an example. However, the seating device 10 in the present application is also usable to build a stiff shoulder prevention system, a low back pain prevention system, and the like that conveys information in real time to people who drive a vehicle for long hours and people who work long hours at a desk.

The lists, the maps, and the graphs generated on the management server 30 in the embodiment described above are an example, and the management server 30 may create charts other than those.

The embodiment described above takes as an example a case in which high pressure alert, insufficient depressurization alert, and high shearing force alert are issued, but may be modified so that other types of alert are issued.

Although not mentioned in the embodiment described above, the seating device 10 may be configured so as to include a cushioning member to be placed on the seat surface X1. In this case, the cushioning member to be used may have a mechanism of varying air pressure in places by taking in or discharging air. When this type of cushioning member is used, the air pressure is automatically adjusted based on the seated body pressure detected by the sensors 14, thereby accomplishing depressurization without requiring the wheelchair user to perform a depressurizing bodily movement himself or herself.

In the embodiment described above, the sensor units 11 including the sensor sheets 13 to which the sensors 14 are affixed and which are covered with the seat covers 15 are used as an example. As another example, a one-sheet sensor may be employed by using a thin film of polyimide or a similar material as one sensor sheet 13, mounting a predetermined number of sensors 14 to the thin film, and covering the thin film and the sensors 14 with one seat cover 15 made of urethane foam or a similar material. In this case, connectors and cords connected to the sensors 14 are contained inside the urethane foam. It is preferred to configure the one-sheet sensor so that a thickness of the one-sheet sensor is approximately from 3 mm to 5 mm.

The thin film may have a thickness of from 0.1 mm to 1.0 mm, and a preferred thickness of the thin film is approximately from 0.1 mm to 0.5 mm. For one sensor sheet 13, a flexible printed circuit board (FPC) obtained by adhering conductive foil such as copper foil to a base film that is a thin film of polyimide or the like may be used.

There are various cushions for the wheelchair X (including a type laid flat on the seat surface X1), such as a type that changes shape depending on air pressure, a type containing a gel-like material inside, a type having a honeycombed internal structure, a type made of urethane foam, and a type having concavities and convexities on a surface. When a sensor in the form of a woven textile such as the sensor as in Patent Literature 1 is laid flat on a cushion that has organized concavities and convexities on a surface, a detection part may fall into space between the concavities and convexities, resulting in a failure to detect the seated body pressure with accuracy. In the case of the sheet-shaped sensor unit 11 described above, on the other hand, the seated body pressure can accurately be detected without the seat surface sensors 14a falling into space between concavities and convexities of a cushion.

When the one-sheet sensor is to be employed, a part placed on the seat surface X1 and a part placed in front of the back support X2 may be integrated into the one-sheet sensor, or separate one-sheet sensors may be used for the former part and the latter part. When the sensor unit 11 is a one-sheet sensor, the sensor unit 11 may be configured so as to be foldable in half in, for example, a direction matching a folding direction of the wheelchair X, which divides the one-sheet sensor into a left half and a right half.

The embodiment described above takes as an example a case in which programs (application programs) for causing the management server 30 to execute the processing procedures described above or processing procedures described later (for causing a computer to function as a server that executes the processing procedures) are stored on the management server 30. Those programs, however, may be stored on a storage medium readable by the management server 30. The term "computer" here means a so-called electronic computer, and is a wide concept encompassing various electronic computers, such as smartphones and tablet terminals in addition to PCs.

In the embodiment described above, a case in which the sensor unit 11 includes both of the seat surface part 11a and the seat back part 11b is taken as an example. When it is sufficient to acquire only seated body pressure data, the seat back part 11b (including the seat back sheet 13b and the seat back cover 15b) may be omitted. When it is sufficient to acquire only back pressure data, the seat surface part 11a (including the seat surface sheet 13a and the seat surface cover 15a) may be omitted.

In the embodiment described above, a case in which a physical therapist registers a new piece of advice is taken as an example. However, a medical doctor, a nurse, a care worker, or the like may register advice on the user terminal 50 used by himself or herself. In this case as well, the same processing as the processing executed to register new advice provided by a physical therapist is executed on the management server 30.

In the embodiment described above, a case in which all processing procedures are executed on the management server 30 is described as an example. However, it is not always required to configure the management server 30 so as to execute all processing procedures, and the management server 30 may be configured so as to execute only required processing. The various types of processing given in the embodiment described above are an example, and the management server 30 may execute processing other than the processing described above. The various types of processing given in the embodiment described above may be executed by hardware, and are also executable by software.

The pressure sore prevention system described above may implement a function of measuring a posture of a seated person from the position of the center of gravity and from the back pressure. Specifically, from a relationship of the position of the center of gravity to pressures on the seat surface X1 and the back support X2, postures such as median, inclined to the left, inclined to the right, turned leftward, turned rightward, left leg crossing, right leg crossing, left back support reclining, and right back support reclining can be predicted. A wheelchair user and any other user of this pressure sore prevention system can view posture information on his or her own user terminal 50.

When this posture measurement function is to be implemented, the management server 30 executes the following processing. For example, when a request to view the posture information is issued from a posture information viewing page displayed on the user terminal 50, the management server 30 reads out data, generates a seated person's posture screen, and transmits the generated seated person's posture screen to the user terminal 50. The seated person's posture screen displays a posture label of, for example, median, inclined to the left, inclined to the right, turned leftward, turned rightward, left leg crossing, right leg crossing, left back support reclining, or right back support reclining.

To which of the median, inclined to the left, inclined to the right, turned leftward, turned rightward, left leg crossing, right leg crossing, left back support reclining, and right back support reclining the seated person's posture corresponds can be determined by, for example, the following method. That is, values of the seated body pressure, the back pressure, and the center-of-gravity position data are combined to set a plurality of conditions, and association between a condition and a posture, such as Condition A and "median," Condition B and "inclined to the left," and Condition C and "inclined to the right," is set in advance. A seated person's posture can be determined by comparing acquired data and the set conditions and determining which of the conditions is satisfied by the seated person's posture. The posture determination method described here is an example, and other methods may be used to determine the posture.

Viewing of the seated person's posture screen displayed on the user terminal 50 of the wheelchair user helps the wheelchair user to set his or her goal in everyday life or behavioral objective, as well as maintain and improve motivation, and can thus lead to a change in behavior of the wheelchair user.

In addition, a primary doctor of the wheelchair user or a physical therapist in charge of the wheelchair user can give advice based on the posture information displayed on the seated person's posture screen, about which aspect is to be improved, what exercise is to be increased, and the like. The advice can be registered by, for example, the steps described in the description of Processing 11 (the new advice registration processing). The wheelchair user can refer to the advice list 92 by the steps described in the description of Processing 6 (the advice list reference processing), and can view the advice by the steps described in the description of Processing 7 (the advice inquiry processing).

The pressure sore prevention system described above may implement a function of enabling the wheelchair user to perform training on his or her own. "Training" here includes sit-ups, back extensions, side bends, rotations, and the like. The training including sit-ups, back extensions, side bends, and rotations is variations executable by the wheelchair user on the wheelchair X.

For example, required training patterns based on a sitting position, seated body pressure data, and the like are set in the management server 30 in advance, and a training pattern is identified by comparing actually acquired data and the sitting position, seated body pressure data, and the like of the set training patterns. The wheelchair user can view the training pattern displayed on the user terminal 50, and execute training of the training pattern.

When this training function is to be implemented, the management server 30 executes the following processing. For example, when a request to view the training information is issued from a training page displayed on the user terminal 50, the management server 30 reads out data, generates training information screen, and transmits the generated training information screen to the user terminal 50. The training information screen displays specific contents to training to be performed.

When the training function is to be implemented, the pressure sore prevention system may be designed so that, for example, a training mode can be set in order to determine whether specified training is performed well from the movement of the position of the center of gravity and other factors. A result of the determination may be used by the physical therapist in charge or other people to determine whether training has appropriately been performed, and to determine contents of subsequent training. The training function is also usable in remote rehabilitation.

The pressure sore prevention system may also implement a function of enabling viewing of comparison information on the user terminal 50. Examples of the comparison information that can be viewed include comparison information for comparing action history of the user of the user terminal 50 himself or herself at any two points in time (in units of days, weeks, months, or other units), and comparison information for comparing the user of the user terminal 50 himself or herself to another person who shares a common matter with the user of the user terminal 50, such as a person belonging to the same age group, a person living in the same geographical area, a person having the same type of disability, or a person engaged in the same occupation. As the comparison information, information such as an average value averaged over a period, or a maximum value or a minimum value in a period is usable.

When this function is to be implemented, the management server 30 executes the following processing. For example, when a request to view the comparison information is issued from a comparison information viewing page displayed on the user terminal 50, the management server 30 reads out data, generates a comparison screen, and transmits the generated comparison screen to the user terminal 50. Viewing of the comparison screen displayed on the user terminal 50 of the wheelchair user helps the wheelchair user to set his or her goal in everyday life or behavioral objective, as well as maintain and improve motivation, and can thus lead to a change in behavior of the wheelchair user.

In addition, a physical therapist in charge of remote rehabilitation can view the comparison information on the user terminal 50 used by the physical therapist when performing remote rehabilitation. The physical therapist in charge of remote rehabilitation can think of a suitable rehabilitation plan and propose the rehabilitation plan to the wheelchair user by viewing, for example, action history information of the wheelchair user at any two points in time.

The pressure sore prevention system may also implement a function of converting a bodily movement of everyday life into a score and presenting an index in the form of a numerical value. For example, a score conversion table in which scores based on the length of time, the number of times, and the like are set for the wheelchair seated time, the seated body pressure, the shearing force, bodily movements, and other items is registered in the management server 30 in advance, actually acquired data is compared to the score conversion table to calculate a score, and the thus calculated score is made available for viewing on the user terminal 50.

When this function is to be implemented, the management server 30 executes the following processing. For example, when a request to view the life score is issued from a life score viewing page displayed on the user terminal 50, the management server 30 reads out data, generates a life score screen, and transmits the generated life score screen to the user terminal 50. Viewing of the life score screen displayed on the user terminal 50 of the user helps the user to set his or her goal in everyday life or behavioral objective, as well as maintain and improve motivation, and can thus lead to a change in behavior of the user.

In addition, a medical service worker, a care manager, a welfare equipment advisor, or the like can, for example, optimize treatment or a care plan by viewing the life score screen displayed on the user terminal 50 used by himself or herself.

The pressure sore prevention system may also implement a function of calculating position information, a travel speed, a road surface condition, and the like, and enabling viewing of those pieces of information on the user terminal 50.

For example, a GPS module (including a communication module that has a GPS function, and the like) may be installed in the seating device 10 to acquire position information data with the GPS module. In this case, the management server 30 may combine the acquired position information data with such information as the seated body pressure, the back pressure, the position of the center of gravity, and whether the user is in the active state, to thereby determine means of travel (whether the user is traveling by car or train, or driving the wheelchair X himself or herself, or by other means), and calculate the distance and the speed of travel by the wheelchair X.

The management server 30 may also use the acquired position information and travel speed, data about the seat surface X1 and the back support X2, and the like to measure travel environment, such as conditions of a road surface (an incline, a rough road, a level difference, and the like) on which the wheelchair user travels, a travel situation (whether mobility is used), and whether there is assistance.

With the implementation of this function, living situations of the wheelchair user as well as problems in living environment encountered by the wheelchair user can be grasped in detail. This function can accordingly be utilized in the so-called smart wellness city initiative.

A local government that wishes to view those pieces of information issues a request to view life information from, for example, a page for viewing life information of wheelchair users which is displayed on the user terminal 50 used by the local government. The management server 30 receives the request, reads out data, generates a life information screen, and transmits the generated life information screen to the user terminal 50 used by the local government. Viewing of the life information screen displayed on the user terminal 50 enables the local government to build a smart wellness city that includes wheelchair users among targets, based on the life information. A significant improvement in lives of wheelchair users, family members of the wheelchair users, people who provide assistance to the wheelchair users, and the like is accordingly expected.

### (Still Another Embodiment)

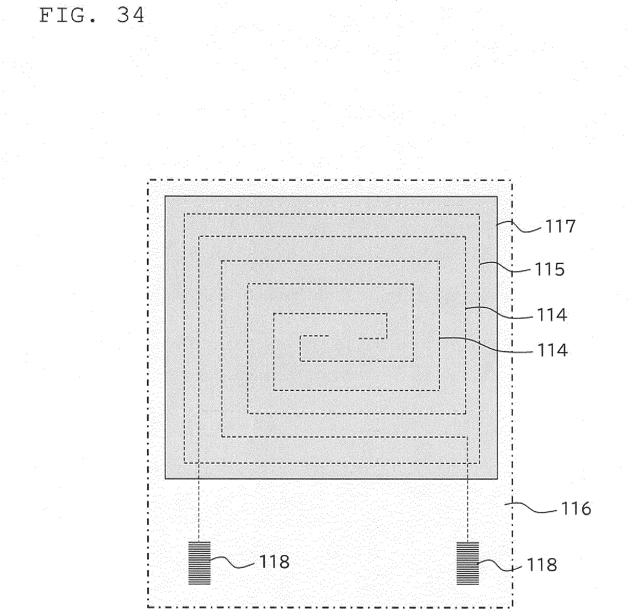

In the pressure sore prevention system of the embodiment described above, a case in which a pressure sensor (see FIG. 3(a) and FIG. 3(b)) that is a type of polymer thick film is used for the sensors 14 is taken as an example. However, other sensors are usable as the sensors 14. For example, a pressure sensor including threads 114 which have conductivity (hereinafter referred to as "conductive threads"), such as the one illustrated in FIG. 34, may be used for the sensors 14.

The sensors 14 illustrated in FIG. 34 include a base cloth 116 to which a resistance cloth 117 is sewn with a sewing thread 115. Two conductive threads 114, which are a positive thread and a negative thread, are sewn to the resistance cloth 117 in a whirlpool pattern. The conductive threads 114 are each run through a bobbin thread (not shown). The pattern into which the conductive threads 114 are sewn is not limited to the whirlpool pattern, and other patterns may be used.

A portion of the base cloth 116 in which the resistance cloth 117 is absent is a conductive wire portion area. One end of each of the conductive threads 114 sewn to the resistance cloth 117 in a whirlpool pattern runs out of the resistance cloth 117 and is sewn to the conductive wire portion area to form an electrode 118. The control unit 12 is connected via the electrode 118 to a bottom wear-side wearable piece 111 and a top wear-side wearable piece 112.

In this embodiment, conductive threads made of polyamide are used as the conductive threads 114, a thread made of polyester is used as the sewing thread 115, a felt cloth material is used as the base cloth 116, and a cloth material made from a carbon mesh cloth is used as the resistance cloth 117. Materials other than those may be used for the conductive threads 114, the sewing thread 115, the base cloth 116, and the resistance cloth 117.

Areas to which the two conductive threads 114, which are a positive thread and a negative thread, are sewn each function as a sensing area. The sensor including the conductive threads 114 is the same as the seat surface sensors 14a and the seat back sensors 14b in that pressure applied to each of the sensors 14 can be specified as an absolute value. The sensing device 100 described here is an example, and an installation-type device other than those described above and a wearable device worn by a wheelchair user (hereinafter referred to as "wearable device") are usable for the sensing device 100.

In the pressure sore prevention system described above, a case in which an installation-type device (the seating device 10) is used for the sensing device 100 is taken as an example. However, a wearable device is also usable for the sensing device 100.

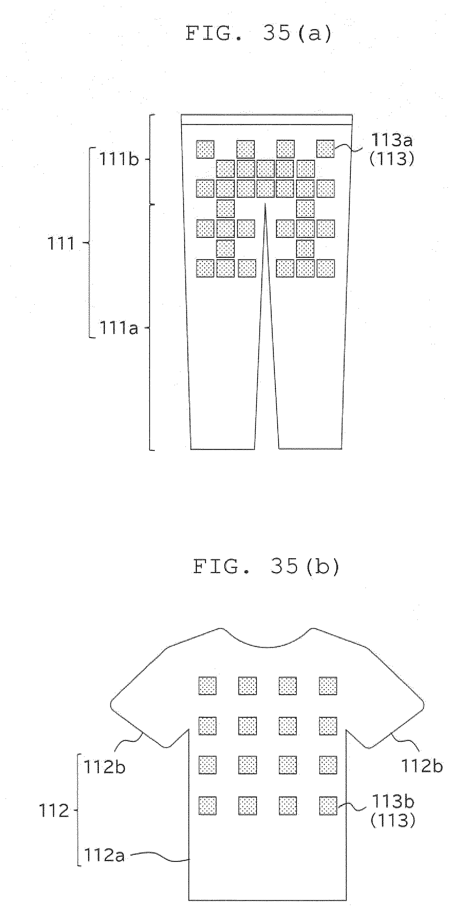

Examples of the wearable device include a bottom wear and a top wear which can be worn by a wheelchair user and to which sensors are mounted, such as the ones illustrated in FIG. 35(a) and FIG. 35(b). "Top wear" is a piece of clothing worn on the upper half of the body, and refers to a so-called top. "Bottom wear" is a piece of clothing worn on the lower half of the body, and refers to a so-called bottom.

The wearable device illustrated in FIG. 35(a) and FIG. 35(b) as an example include the bottom wear-side wearable piece 111 (FIG. 35(a)) to be worn on the lower half of the body, and the top wear-side wearable piece 112 (FIG. 35(b)) to be worn on the upper half of the body. The bottom wear-side wearable piece 111 in this embodiment is shaped like a pair of trousers, and includes two leg covering portions 111a in which legs of the wearer are fit, and a hip covering portion 111b in which a portion of the body above the legs and around the hips are fit.

Sensors 113 (hereinafter referred to as "bottom wear-side sensors 113a") corresponding to the seat surface sensors 14a of the seating device 10 described above are provided in areas (femoral areas) of the leg covering portions 111a that are on the back side of the femoral areas, and in an area (gluteal area) of the hip covering portion 111b that is on the back side of the buttocks. In an area in which a cloth material forming the bottom wear-side wearable piece 111 is present, a no-sensor area corresponding to the no-sensor area 14c of the seating device 10 described above is provided.

As is the case for the seating device 10 described above, the number of bottom wear-side sensors 113a can be minimized by providing an area in which the cloth material forming the bottom wear-side wearable piece 111 is present with the no-sensor area in which the bottom wear-side sensors 113a are not provided. Consequently, a time required for transmission of data to the management server 30 (external equipment) can be shortened, and a system capable of conveying information to a wheelchair user in real time can be built.

Portions of the bottom wear-side wearable piece 111 other than the femoral area and the gluteal area can also be treated as non-sensor areas in which the sensors 113 are not provided. The no-sensor areas in this case include a space portion between the leg covering portions 111a in which the cloth material forming the bottom wear-side wearable piece 111 is not present.

The top wear-side wearable piece 112 in this embodiment is shaped like a T-shirt, and includes a trunk portion 112a and two sleeve portions 112b. A back side (back area) of the trunk portion 112a is provided with the sensors 113 (hereinafter referred to as "top wear-side sensors 113b") corresponding to the seat back sensors 14b of the seating device 10 described above.

A pressure sensor that is a type of polymer thick film such as the ones illustrated in FIG. 3(a) and FIG. 3(b), a pressure sensor including the conductive threads 114 such as the one illustrated in FIG. 34, or the like is usable for the bottom wear-side sensors 113a and the top wear-side sensors 113b. When a pressure sensor including the conductive threads 114 is used for the bottom wear-side sensors 113a and the top wear-side sensors 113b, the cloth material forming the bottom wear-side wearable piece 111 and the top wear-side wearable piece 112 can be used as the base cloth 116.

The present invention is not limited to the structures and shapes of the embodiments described above, and are receptive of various modifications and changes without departing from the spirit of the invention. For example, the present invention is applicable to an information processing system other than the pressure sore prevention system described above. In this case, the information processing system may include components different from the components of the pressure sore prevention system described above. The management server 30 may have a configuration designed to suit the information processing system to be built as well. The functions implemented by the management server 30 may be distributed among a plurality of servers, or may be aggregated in a single server. It is sufficient for the information processing system to function on the whole, and which function is implemented by which apparatus can be designed to suit the information processing system to be built. The configurations of the screens to be displayed are an example, and the screens may have configurations different from those in the embodiments described above.

### Industrial Applicability

The information processing system, the management server, the program, and the storage medium according to the present invention are usable to build, in addition to a system for preventing a pressure sore of a wheelchair user, for example, a system for preventing stiff shoulder and low back pain of people who sit on a chair or other seats for a long stretch of time, such as people who drive a vehicle for long hours and people who work long hours at a desk.

### Reference Signs List

- 10: seating device
- 11: sensor unit
- 11a: seat surface part
- 11b: seat back part
- 12: control unit
- 13: sensor sheet
- 13a: seat surface sheet
- 13b: seat back sheet
- 14: sensor
- 14a: seat surface sensor
- 14b: seat back sensor
- 14c: no-sensor area
- 15: seat cover
- 15a: seat surface cover
- 15b: seat back cover
- 16: microcomputer
- 16a: microcomputer board
- 16b: electronic part
- 17: battery
- 18: communication module
- 19: storage box
- 30: management server (external equipment)
- 31: processor
- 32: memory
- 33: storage
- 34: communication part
- 34a: reception part
- 34b: transmission part
- 35: input part
- 36: output part
- 37: bus
- 300: Web server
- 301: request reception part
- 302: processing request part
- 303: result reception part
- 304: response screen generation part
- 320: application server (AP server)
- 321: pre-processing part
- 322: request reception part
- 323: user registration part
- 324: chart generation part
- 325: alert determination part
- 326: action determination part
- 327: advice reception part
- 328: reservation reception part
- 340: database server (DB server)
- 341: user information storage part
- 342: sensor information storage part
- 343: alert information storage part
- 344: advice information storage part
- 345: set condition storage part
- 50: user terminal
- 51: processor
- 52: memory
- 53: storage
- 54: communication part
- 54a: reception part
- 54b: transmission part
- 55: input part
- 56: output part
- 57: bus
- 60: login screen
- 60a: ID input field
- 60b: password input field
- 60c: "login" button
- 61: main screen
- 61a: main menu
- 61b: "register user information" button
- 61c: "refer to data" button
- 61d: "advice" button
- 61e: "alert" button
- 62: user information registration screen
- 62a: user information input portion
- 62b: "register" button
- 63: data reference screen
- 63a: "real time" tab
- 63b: "period specified" tab
- 63c: "start recording" button
- 63d: "end recording" button
- 63e: "save" button
- 63f: offset input portion
- 63g: record list display portion
- 63h: "play" button
- 63i: "stop" button
- 63j: simple specification portion
- 63k: details specification portion
- 63m: "update" button
- 63n: "settings" button
- 64: action inquiry screen
- 64a: action graph display portion
- 64b: inquiry condition input portion
- 64c: "update" button
- 64d: period specification portion
- 64e: unit selection portion
- 64f: display item selection portion
- 65: setting screen
- 65a: site setting portion
- 65b: action goal setting portion
- 65c: action detection reference setting portion
- 65d: alert reference setting portion
- 65e: "enter" button
- 65f: row display block
- 65g: column display block
- 66: advice list screen
- 66a: search criteria input portion
- 66b: "new advice" button
- 66c: advice list display portion
- 66d: "search" button
- 67: advice inquiry screen
- 67a: basic information display portion
- 68: new advice input screen
- 68a: condition setting portion
- 68b: advice input portion
- 68c: "enter" button
- 68d: list display portion
- 68e: registration confirmation screen
- 68f: "register" button
- 69: alert list screen
- 69a: search criteria input portion
- 69b: alert list display portion
- 69c: "search" button
- 70: alert inquiry screen
- 70a: basic information display portion
- 70b: "input advice" button
- 70c: "return" button
- 70d: list display portion
- 81: heat map (seat surface heat map)
- 81a: center-of-gravity point
- 81b: checkbox of "display shearing force"
- 82: heat map (seat back heat map)
- 83: shearing force map
- 84: shearing force graph
- 85: seated body pressure graph
- 86: action graph
- 87: setting map
- 91: bodily movement item list
- 92: advice list
- 93: alert list
- 94: record list
- 100: sensing device
- 111: bottom wear-side wearable piece
- 111a: leg covering portion
- 111b: hip covering portion
- 112: top wear-side wearable piece
- 112a: trunk portion
- 112b: sleeve portion
- 113: sensor
- 113a: bottom wear-side sensor
- 113b: top wear-side sensor
- 114: conductive thread
- 115: sewing thread
- 116: base cloth
- 117: resistance cloth
- 118: electrode
- A1: first quadrant
- A2: second quadrant
- A3: third quadrant
- A4: fourth quadrant
- B1: left-side area
- B2: right-side area
- B3: front-side area
- B4: back-side area
- L1: front-back division line
- L2: left-right division line
- N: communication network
- X: wheelchair (seat)
- X1: seat surface (of wheel chair)
- X2: back support (of wheel chair)

## Claims

1. An information providing system for providing information to a seat user via a communication network,
the information providing system comprising a management server provided in a cloud, a sensing device configured to acquire data about a seating situation of the seat user and transmit the data to the management server, a seat user terminal to be used by the seat user, and an advice provider terminal to be used by an advice provider, the management server, the sensing device, the seat user terminal, and the advice provider terminal being connected to one another via the communication network,
wherein the management server includes a communication part configured to hold data communication via the communication network, and an advice reception part configured to receive advice transmitted from the advice provider terminal, and
wherein the advice is received by the advice reception part and is then transmitted to the seat user terminal via the communication part.

2. The information providing system according to claim 1,
wherein the management server further includes an alert determination part configured to determine, based on the data about the seating situation which is transmitted from the sensing device, whether the seating situation of the seat user satisfies an alert condition set in advance, and
wherein, when the alert determination part determines that the seating situation of the seat user satisfies the alert condition set in advance, alert is transmitted to the seat user terminal via the communication part.

3. The information providing system according to claim 1 or 2, wherein the alert and the advice are transmitted via the communication part by an SNS, an SMS, or an electronic mail that are viewable on the seat user terminal.

4. The information providing system according to any one of claims 1 to 3, wherein the management server further includes a chart generation part configured to generate charts based on the data about the seating situation which is transmitted from the sensing device.

5. The information providing system according to claim 4, wherein the chart generation part is configured to generate a seat surface heat map on which pressure values of a seat surface part detected by seat surface sensors of the sensing device and interpolation values calculated from the pressure values are displayed in colors that vary depending on magnitudes of values.

6. The information providing system according to claim 5,
wherein the sensing device includes the seat surface sensors and seat back sensors, and
wherein the chart generation part is configured to generate a shearing force map for displaying, as a vector, a shearing force calculated from pressure values of the seat surface part which are acquired by the seat surface sensors and pressure values of a seat back part which are acquired by the seat back sensors.

7. The information providing system according to claim 6, wherein the shearing force map is displayed alone or overlaid on the seat surface heat map.

8. The information providing system according to any one of claims 1 to 7, wherein the management server further includes an action determination part configured to determine, based on the data about the seating situation which is transmitted from the sensing device, whether the seat user is executing an action set in advance.

9. The information providing system according to any one of claims 1 to 8, wherein the management server further includes a reservation reception part configured to receive a reservation for remote rehabilitation which is transmitted from the seat user terminal.

10. The information providing system according to any one of claims 1 to 9,
wherein the sensing device includes a sensor unit,
wherein the sensor unit includes a seat surface part placed on a seat surface of the seat,
wherein a femoral area and a gluteal area of the seat surface part have seat surface sensors provided therein, and
wherein the femoral area and the gluteal area have provided therebetween a no-sensor area in which no seat surface sensors are installed.

11. A management server, which is provided in a cloud to provide information to a seat user via a communication network, the management server comprising:
a communication part configured to hold data communication via the communication network; and
an advice reception part configured to receive advice transmitted from an advice provider terminal, which is connected via the communication network,
wherein the advice is received by the advice reception part and is then transmitted, via the communication part, to a seat user terminal connected via the communication network.

12. The management server according to claim 11, further comprising an alert determination part configured to determine, based on data about a seating situation which is transmitted from a sensing device, whether the seating situation of the seat user satisfies an alert condition set in advance,
wherein, when the alert determination part determines that the seating situation of the seat user satisfies the alert condition set in advance, alert is transmitted to the seat user terminal via the communication part.

13. The management server according to claim 11 or 12, wherein the advice and the alert are transmitted via the communication part by an SNS, an SMS, or an electronic mail that are viewable on the seat user terminal.

14. The management server according to any one of claims 11 to 13, further comprising a chart generation part configured to generate charts based on the data about the seating situation which is transmitted from the sensing device.

15. The management server according to claim 14, wherein the chart generation part is configured to generate a seat surface heat map on which pressure values of a seat surface part detected by seat surface sensors of the sensing device and interpolation values calculated from the pressure values are displayed in colors that vary depending on magnitudes of values.

16. The management server according to claim 15,
wherein the sensing device includes the seat surface sensors and seat back sensors, and
wherein the chart generation part is configured to generate a shearing force map for displaying, as a vector, a shearing force calculated from pressure values of the seat surface part which are acquired by the seat surface sensors and pressure values of a seat back part which are acquired by the seat back sensors.

17. The management server according to claim 16, wherein the shearing force map is displayed alone or overlaid on the seat surface heat map.

18. The management server according to any one of claims 11 to 17, further comprising an action determination part configured to determine, based on the data about the seating situation which is transmitted from the sensing device, whether the seat user is executing an action set in advance.

19. The management server according to any one of claims 11 to 18, further comprising a reservation reception part configured to receive a reservation for remote rehabilitation which is transmitted from the seat user terminal.

20. A program for causing a computer to function as the management server of any one of claims 11 to 19.

21. A computer-readable storage medium having the program of claim 20 recorded thereon.
